# EUROPEAN PATENT SPECIFICATION

(11) **EP 3 388 014 B1**
(45) Date of publication and mention of the grant of the patent: **01.03.2023**
(21) Application number: 16872609.9
(22) Date of filing: 30.11.2016
(51) Int. Cl.: A61B 17/00, A61B 90/00, A61B 34/37, B25J 9/08

(54) **SURGICAL SYSTEM**
CHIRURGISCHES SYSTEM
SYSTÈME CHRIRURGICALE

(30) Priority: 11.12.2015 JP 2015242698
(43) Date of publication of application: 17.10.2018
(73) Proprietor: Kawasaki Jukogyo Kabushiki Kaisha, Kobe-shi, Hyogo 650-8670 (JP)
(72) Inventor: NAKANISHI, Tetsuya, Kobe-shi, Hyogo 650-8670 (JP)
(74) Representative: Müller-Boré & Partner Patentanwälte PartG mbB
(86) International application number: PCT/JP2016/005033
(87) International publication number: WO 2017/098707

(56) References cited:
- WO-A1-00/33755
- WO-A1-2006/079108
- WO-A1-2014/146085
- WO-A1-2015/175218
- WO-A2-2010/068005
- JP-A- 2002 361 578
- JP-A- 2004 208 922
- JP-A- 2008 528 130
- JP-A- 2010 214 166
- JP-A- 2015 530 906
- US-A1- 2003 208 189
- US-A1- 2004 261 179
- US-B1- 6 331 181

## Description

### Technical Field

The present invention relates to a manipulator arm to which an instrument for performing a surgical operation is attached, a manipulator arm support body, and a surgical system including them.

### Background Art

Conventionally, a surgical operation using a surgical system including a manipulator, such as a robot-assisted surgical operation or a remote robot surgical operation, has been known. This system includes instruments including surgical instruments such as forceps and scalpels and endoscope cameras and manipulator arms having the instruments attached to the distal end portions. The operator remotely controls the operation of a manipulator arm and an instrument by using the manipulation device installed in a place away from a patient, thereby performing an operation on a surgical site of the patient. This type of surgical system is disclosed in PTLs 1 and 2.

The surgical system (robot surgical system) disclosed in PTL 1 includes a platform, a plurality of setup arms coupled to the platform, a manipulator base coupled to the distal end part of each setup arm, a manipulator coupled to the manipulator base, and a surgical instrument attached to the distal end part of the manipulator. The platform is vertically movably suspended from the ceiling of an operating room via a platform linkage provided on the ceiling of the operating room.

In addition, PTL 2 discloses a surgical robot system for carrying out a surgical operation in a sterile field. This robot system includes a robot arm, a wrist unit provided at the distal end part of the robot arm, and a surgical instrument detachably attached to the wrist unit. The drive assembly of a robot arm is provided at the proximal end part of the robot arm. This drive assembly is housed in an attachment bracket. This attachment bracket is connected to an attachment joint supported by an attachment arm on which a rail provided on a side of an operating table can travel. The robot arm and wrist unit are then covered with a sterile drape that shields these non-sterile parts from the sterile field.
WO 2015/175218 A1 describes a surgical robotic arm support system including a base and at least one post and/or a connection for coupling a robot arm to the support system. The base may be configured to at least partially surround a surgical table. The at least one post may have a first end supported on the base and a second end configured to support a robotic arm. The at least one post may be configured to be coupled to a surgical table. The connection may support a mechanical and/or electrical connectivity between the support system and a coupled robot arm. Electrical connectivity may be configured to identify at least one of the robot arm connections to which at least one robot arm is connected. Each robot arm connection may be configured to maintain a portion of a connected robot arm at a predetermined position from the base.
WO 2006/079108 A1 describes a robotic surgery system comprising a mounting base, a plurality of surgical instruments, and an articulate support assembly Each instrument is insertable into a patient through an associated minimally invasive aperture to a desired internal surgical site. The articulate support assembly movably supports the instruments relative to the base. The support generally comprises an orienting platform, a platform linkage movably supporting the orienting platform relative to the base, and a plurality of manipulators mounted to the orienting platform, wherein each manipulator movably supports an associated instrument.
WO 2010/068005 A2 describes a surgical robot. A bed mount surgical robot, for performing a surgical procedure on a patient lying on an operating bed using a surgical instrument mounted on an end portion of a robot arm, can include: a main unit joined to the operating bed, a support arm rotatably joined to the main unit, and one or more robot arms rotatably joined to the support arm. Since multiple robot arms may be joined to a support arm, which itself may be joined to a main unit mounted on the operating bed, the surgical slave robot can have a slim, compact form as well as greater stability. Also, as the robot arms may be mounted onto the bed, the relative positions of the robot arms with respect to the bed can be accurately identified without having to separately input the position information of the bed when installing the robot arms.
WO 00/33755 A1 describes robotic surgical tools, systems, and methods for preparing for, and performing robotic surgery include a memory mounted on the tool. The memory can perform a number of functions when the tool is loaded on the tool manipulator. First, the memory can provide a signal verifying that the tool is compatible with that particular robotic system. Secondly, the tool memory may identify the tool-type to the robotic system so that the robotic system can reconfigure its programming. Thirdly, the memory of the tool may indicate tool specific information, including measured calibration offsets indicating misalignment of the tool drive system, tool life data, or the like. This information may be stored in a read only memory (ROM), or in a nonvolatile memory which can be written to only a single time. The invention further provides improved engagement structures for coupling robotic surgical tools with manipulator structures.

### Citation List

### Patent Literature

PTL 1: JP 2012-143589 A
PTL2: JP 2015-62734 A

### Summary of Invention

### Technical Problem

In the configuration disclosed in PTL 1, a plurality of setup arms are provided, and one manipulator is supported by each setup arm. In addition, in the configuration disclosed in PTL 2, a plurality of attachment joints are provided on an operation table, and the attachment bracket of one robot arm is attached to each attachment joint. In the configurations disclosed in PTLs 1 and 2, an instrument is detachable from a manipulator (or a robot arm), but a manipulator (or a robot arm) is coupled to a setup arm (or an attachment bracket) so as not to be easily detached from it.

### Solution to Problem

The number, types, combination, and the like of instruments used during surgery vary depending on the type and contents of surgery. For example, three or more instruments or three or more types of instruments are simultaneously used in some case, and two or more instruments or two or more types of instruments are simultaneously used in another case. In the former case, at least four manipulator arms including the manipulator arm to which an endoscope camera is attached are necessary. In the latter case, three manipulator arms are sufficient. Therefore, in the latter case, some manipulator arms are sometimes not used during surgery. In addition, for example, depending on the contents of surgery (the position of an affected part), a plurality of manipulator arms provided in a surgical system are sometimes required to have different lengths.

In view of the above circumstances, the present inventors have conceived to customize the number, types, combination, and the like of manipulator arms in a surgical system including manipulators before and/or during surgery. This makes it possible to construct a proper surgical system for each operation.

Accordingly, the present invention provides, in a surgical system including manipulators, a technique for easily customizing the configuration of each manipulator arm supporting an instrument in accordance with the contents of surgery before and/or during the surgery, as defined in independent claim 1.

A surgical system according to one aspect includes a plurality of instruments for performing a surgical operation, a platform, a plurality of manipulator arms having proximal end portions detachably attached to the platform and distal end portions, to each of which one of the plurality of instruments is detachably attached, and configured to movably support the instrument, and an attachment lock mechanism configured to lock and unlock each manipulator arm attached to the platform.

A manipulator arm according to one aspect is a manipulator arm which is attached to a support body and has a distal end portion to which an instrument for performing a surgical operation is attached. The manipulator arm includes a base, at least one link coupled in series with the base, and an interface unit provided at the proximal end portion of the base and having an arm side engagement portion configured to be engaged with a support body side engagement portion provided on the support body. When the support body side engagement portion is engaged with the arm side engagement portion, the interface unit is locked while being attached to the support body. When the support body side engagement portion is disengaged from the arm side engagement portion, the interface unit is unlocked so as to be detached from the support body.

A manipulator arm support body according to one aspect includes a platform to which a manipulator arm configured to movably support an instrument for performing a surgical operation is attached, and a positioner configured to movably support the platform. The platform includes a plurality of manipulator arm attachment portions to each of which the manipulator arm is detachably attached and a support body side engagement portion configured to be engaged with an arm side engagement portion provided on the manipulator arm. When the support body side engagement portion is engaged with the arm side engagement portion, the manipulator arm is locked while being attached to the manipulator arm attachment portion. When the support body side engagement portion is disengaged from the arm side engagement portion, the manipulator arm is unlocked so as to be detached from the manipulator arm attachment portion. Note that, in the surgical system, the manipulator arm, and the manipulator arm support body, the instrument may be an endoscope camera assembly.

In the surgical system, the manipulator arm, and the manipulator arm support body, the manipulator arm is disposed on the platform and is disposed and locked by the attachment lock mechanism (the support body side engagement portion and the arm side engagement portion), thereby attaching the manipulator arm to the platform. In addition, by unlocking the manipulator arm attached to the platform, it is possible to detach the manipulator arm from the platform. In this way, the manipulator arm is detachable from the platform, and the manipulator arm attached to the platform can be replaced or the manipulator arm attached to a certain attachment position of the platform can be replaced to another attachment position before and/or during surgery. In this manner, before and/or during surgery, the system can be properly customized for the surgery by attaching manipulator arms matching the number, type, and combination set in accordance with the contents of surgery to the platform.

### Advantageous Effects of Invention

The present invention can provide, in a surgical system including manipulators, a technique for easily customizing the configuration of each manipulator arm supporting an instrument in accordance with the contents of surgery before and/or during the surgery as defined in independent claim 1. Preferred embodiments are disclosed in the dependent claims.

### Brief Description of Drawings

Fig. 1 is a schematic diagram showing the overall configuration of a surgical system according to an embodiment of the present invention.
Fig. 2 is a side view showing the overall configuration of a positioner.
Fig. 3 is a block diagram showing a schematic configuration of the control system of the positioner.
Fig. 4 is a side view showing the overall configuration of the positioner with its swing arm being tilted from a vertical position.
Fig. 5 is a side view showing the overall configuration of the positioner with its platform being tilted from a horizontal position.
Fig. 6 is a side view showing the overall configuration of the positioner when a columnar member is provided instead of a lifting shaft.
Fig. 7 is a schematic diagram showing the overall configuration of a patient-side manipulator arm.
Fig. 8 is a block diagram showing a schematic configuration of the control system of an arm body.
Fig. 9 is a partial cross-sectional view of the arm body, showing a layout of a drive system of the arm body.
Fig. 10 is a plan view showing a coupling structure between a platform and the patient-side manipulator arm.
Fig. 11 is a cross-sectional view taken along a line XI-XI in Fig. 10.
Fig. 12 is a block diagram showing a configuration for managing the patient-side manipulator arm attached to the platform.
Fig. 13 is a plan view showing a coupling structure between the platform and the patient-side manipulator arm when a servomotor is provided on the platform.
Fig. 14 is a partially cutaway view showing a configuration example of the distal end portion of the arm body and a translation arm of the surgical system in Fig. 1.
Fig. 15 is a view showing an operation example of the translation arm.
Fig. 16 is a diagram showing a configuration example of a swing mechanism.
Fig. 17 is a diagram showing an operation example of the swing mechanism.
Fig. 18 is a view showing the first modification of the translation arm.
Fig. 19 is a view showing the second modification of the translation arm.
Fig. 20 is a view showing the first modification of the swing mechanism.
Fig. 21 is a view showing the second modification of the swing mechanism.
Fig. 22 is a view showing the first modification of each arm including the swing mechanism.
Fig. 23 is a view showing the second modification of each arm including the swing mechanism.

A surgical system according to an embodiment includes a plurality of instruments for performing a surgical operation, a platform, a plurality of manipulator arms having proximal end portions detachably attached to the platform and distal end portions, to each of which one of the plurality of instruments is detachably attached, and configured to movably support the instruments, and an attachment lock mechanism configured to lock and unlock each manipulator arm attached to the platform.

In the above surgical system, the platform may have more manipulator arm attachment portions than the plurality of manipulator arms.

The surgical system may further include a positioner that movably supports the platform.

The surgical system may further include a detection sensor for detecting the presence or absence of the manipulator arm attached to the platform and a management device for detecting that the manipulator arm is attached to the platform based on a detection signal from the detection sensor.

In the above surgical system, a reader configured to read out information held by the manipulator arm is provided for the platform. Upon detecting attachment of the manipulator arm to the platform, the management device may be configured to cause the reader to read out the information, acquire the information read out by the reader, and temporarily store the acquired information in association with the attachment position on the platform.

In the above surgical system, the information held by the manipulator arm includes the individual identification information of the manipulator arm, and surgical information including a combination of a plurality of manipulator arms used in surgery is set in the management device in advance. The management device may be configured to output a warning if a combination of individual identification information included in information acquired from the reader does not correspond to the surgery information.

In the above surgical system, the information held by the manipulator arm includes the individual identification information of the manipulator arm, and surgical information including a combination of the individual identification information of each manipulator arm used in surgery and an attachment position on the platform to which the manipulator arm is to be attached is set in the management device in advance. The management device may be configured to output a warning if a combination of individual identification information included in information acquired from the reader and the attachment position on the platform does not correspond to the surgery information.

In the above surgical system, the information held by the manipulator arm includes model number information of the manipulator arm, and surgical information including a combination of the model number information of each manipulator arm used in surgery and an attachment position on the platform to which the manipulator arm is to be attached is set in the management device in advance. The management device may be configured to output a warning if a combination of model number information included in information acquired from the reader and the attachment position on the platform does not correspond to the surgery information.

In the above surgical system, the information held by the manipulator arm includes the individual identification information of the manipulator arm, and surgical information including a combination of the model number information of each manipulator arm to be used in surgery and the attachment position on the platform to which the manipulator arm is to be attached is set in the management device in advance. The management device may be configured to have a model number storage unit storing the model number information of each manipulator arm associated with individual identification information, read out corresponding model number information from the model number storage unit, based on the individual identification information included in the information acquired from the reader, and output a warning if a combination of the readout model number information and an attachment position on the platform does not correspond to the surgery information.

In the above surgical system, the information held by the manipulator arm includes the individual identification information and usage count information of the manipulator arm, and the management device may be configured to have a usage limit number storage unit storing a usage limit number associated with the individual identification information, read out the usage limit number of the manipulator arm from the usage limit number storage unit based on the individual identification information included in the information acquired from the reader, and output a warning if the usage count information included in the information acquired from the reader exceeds the usage limit number.

In the above surgical system, the information held by the manipulator arm is stored in an IC tag provided for the manipulator arm, the reader has a function of writing to the IC tag, and the management device may be configured to cause the reader to operate so as to write, in the IC tag, new usage count information obtained by adding 1 to the usage count information acquired from the reader.

In the above surgical system, a socket of an electric wire or communication wiring extending through the platform is provided for the platform. When the manipulator arm is attached to the platform at the proximal end portion of the manipulator arm, a connector connected to an electric wire or communication wiring of the manipulator arm may be provided at a position where the connector is connected to the socket.

In the above surgical system, a motor is provided for the platform, and an input shaft to the power system of the manipulator arm is provided at the proximal end portion of the manipulator arm. When the proximal end portion of the manipulator arm is attached to the platform, a shaft coupling that connects the output shaft of the motor and the input shaft so as to allow power transmission may be provided at an end portion of the output shaft and an end portion of the input shaft.

In the above surgical system, the platform, the manipulator arm, and the instrument are installed in a sterile field, and the platform may be shielded from the sterile field by a sterile drape.

A manipulator arm according to an embodiment of the present invention described below is a manipulator arm which is attached to a support body and has a distal end portion to which an instrument for performing a surgical operation is attached. The manipulator arm includes a base, at least one link coupled in series with the base, and an interface unit provided at the proximal end portion of the base and having an arm side engagement portion configured to be engaged with a support body side engagement portion provided on the support body. When the support body side engagement portion is engaged with the arm side engagement portion, the interface unit is locked while being attached to the support body. When the support body side engagement portion is disengaged from the arm side engagement portion, the interface unit is unlocked so as to be detached from the support body.

The manipulator arm may have information readable by a reader provided on the support body side.

In the manipulator arm, the information may be individual identification information.

In the manipulator arm, the information may be model number information.

In the manipulator arm, the information may be individual identification information and usage count information.

In the manipulator arm, the information may be stored in an IC tag provided for the interface unit.

In the above manipulator arm, a connector for connecting the wiring passed through the support body and the wiring passed through the manipulator arm may be provided for the interface unit.

In the manipulator arm, the interface unit may be provided with an input unit for receiving an input from a power source provided on the support body.

A manipulator arm support body according to an embodiment of the present invention described below includes a platform to which a manipulator arm configured to movably support an instrument for performing a surgical operation is attached, and a positioner configured to movably support the platform. The platform includes a plurality of arm attachment portions to which the manipulator arms are detachably attached and a support body side engagement portion configured to be engaged with an arm side engagement portion provided on the manipulator arm. When the support body side engagement portion is engaged with the arm side engagement portion, the manipulator arm is locked while being attached to the arm attachment portion. When the support body side engagement portion is disengaged from the arm side engagement portion, the manipulator arm is unlocked so as to be detached from the arm attachment portion.

The above surgical system and the above manipulator arm will be described in detail with reference to the accompanying drawings.

### [Outline of Surgical System]

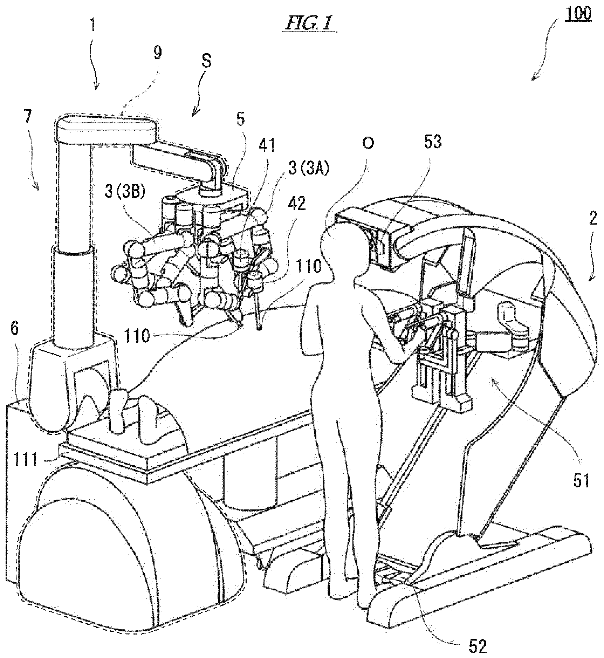

Fig. 1 is a schematic diagram showing the overall configuration of a surgical system 100 according to an embodiment of the present invention. As shown in Fig. 1, the surgical system 100 is a system that is used by an operator O such as a doctor to perform an endoscopic surgical operation on a patient P using a patient-side system 1 as in the case of robot-assisted surgery or robot remote surgery.

The surgical system 100 includes the patient-side system 1 and an operation device 2 for manipulating the patient-side system 1. The operation device 2 is disposed away from the patient-side system 1, and the patient-side system 1 is remotely operated by the operation device 2. The operator O inputs information concerning the operation to be performed by the patient-side system 1 to the operation device 2, and the operation device 2 transmits the corresponding operation command to the patient-side system 1. The patient-side system 1 then receives the operation command transmitted from the operation device 2, and causes an endoscope assembly 41, an instrument 42 (surgical instrument), or the like of the patient-side system 1 to operate based on this operation command. Each component of the surgical system 100 will be described in detail below.

### [Configuration Example of Operation Device]

The operation device 2 is a device that constitutes an interface between the surgical system 100 and the operator O and operates the patient-side system 1. The operation device 2 is disposed beside an operating table 111 or away from the operating table 111 in an operating room or outside the operating room. The operation device 2 includes an operation input unit 50 including an operation manipulator arm 51 and an operation pedal 52 with which the operator O inputs an operation command and a monitor 53 that displays an image photographed by the endoscope assembly 41. While visually checking an affected part with the monitor 53, the operator O operates the operation input unit 50 to input an operation command to the operation device 2. The operation command input to the operation device 2 is transmitted to a controller 6 (described later) of the patient-side system 1 wiredly or wirelessly

### [Configuration Example of Patient-Side System]

The patient-side system 1 constitutes an interface between the surgical system 100 and a patient P. The patient-side system 1 is disposed beside the operating table 111 on which the patient P lies in the operating room. The inside of the operating room is a sterilized sterile field.

The patient-side system 1 includes a positioner 7, a platform 5 attached to the distal end of the positioner 7, a plurality of patient-side manipulator arms (to be simply referred to as "arms 3" hereinafter) detachably attached to the platform 5, the endoscope assembly 41 attached to the distal end portion of one arm 3A of the plurality of arms 3, the instrument 42 detachably attached to the distal end portion of a remaining arm 3B of the plurality of arms 3, a sterile drape 9 for shielding the positioner 7 and the platform 5 from the sterile field, and the controller 6 for controlling the operation of the patient-side system 1. The arm 3 to which the endoscope assembly 41 is attached is sometimes referred to as "camera arm 3A", and the arm 3 to which the instrument 42 is attached is sometimes referred to as "instrument arm 3B". The patient-side system 1 according to this embodiment includes four arms 3 including one camera arm 3A and three instrument arms 3B.

In the patient-side system 1, the platform 5 has a function as a "hub" serving as the base of the plurality of arms 3. In this embodiment, the positioner 7 and the platform 5 constitute a manipulator arm support body S that movably supports the plurality of arms 3. However, the manipulator arm support body S may include at least the platform 5. For example, the manipulator arm support body S may be formed from the platform 5 supported by a direct-acting rail, a lifting device, or the platform 5 supported on a bracket attached to a ceiling or wall instead of the positioner 7.

In the patient-side system 1, the elements ranging from the positioner 7 to the endoscope assembly 41 or each instrument 42 are continuously coupled to each other. In this specification, the end portion of each of the above-described series of elements which is located on the side facing the positioner 7 (more specifically, the contact portion of the positioner 7 with the floor of the operating room) will be referred to as a "proximal end portion", and the end portion on the opposite side will be referred to as a "distal end portion". The proximal end portion is sometimes referred to as the "proximal end part", and the distal end portion is sometimes referred to as the "distal end part".

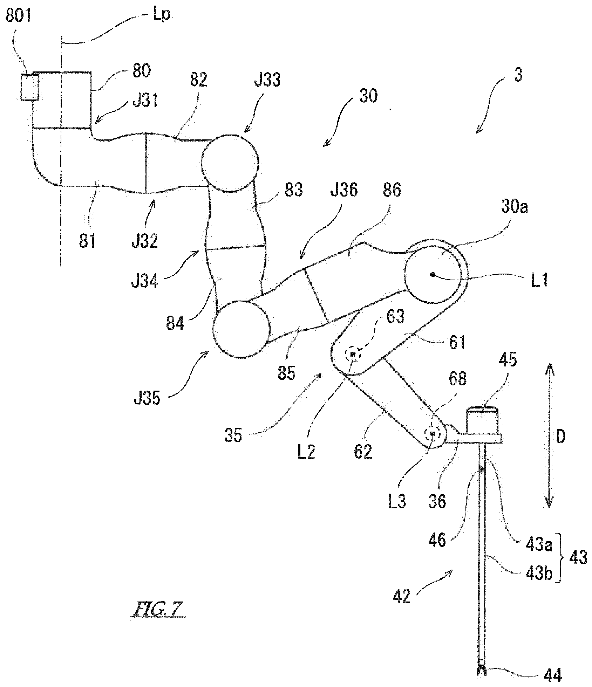

The instrument 42 is constituted by a drive unit 45 provided at its proximal end portion, an end effector 44 (treatment tool) provided at the distal end portion, an elongated shaft 43 coupling the drive unit 45 and the end effector 44 (see Fig. 7 for each component). A reference direction D is defined for the instrument 42, and the drive unit 45, the shaft 43, and the end effector 44 are aligned in parallel with the reference direction D. The end effector 44 of the instrument 42 is selected from the group consisting of instruments having joints designed to operate (e.g., forceps, scissors, grasper, needle holder, microdissector, staple applier, tucker, suction cleaning tool, snare wire, clip applier) and instruments without joints (e.g., a cutting blade, ablation probe, scrubbers, catheter, and suction orifices).

In the patient-side system 1 having the above-described configuration, the controller 6 having received an operation command from the operation device 2 firstly operates the positioner 7 so as to make the platform 5 and the operating table 111 or the patient P have a predetermined positional relationship, thereby positioning the platform 5. Next, the controller 6 operates each arm 3 to position the endoscope assembly 41 and the respective instruments 42 such that a sleeve (cannula sleeve) 110 indwelled in the body surface of the patient P, the endoscope assembly 41, and each instrument 42 have a predetermined initial positional relationship. The above positioning operations of the positioner 7 and each arm 3 may be performed at the same time. In a state in which the positioner 7 is kept stationary in principle, the controller 6 operates each arm 3 in accordance with an operation command from the operation device 2 to operate each instrument 42 so as to perform a medical procedure while appropriately displacing the endoscope assembly 41 and the instrument 42 and changing their postures.

### [Configuration Example of Positioner]

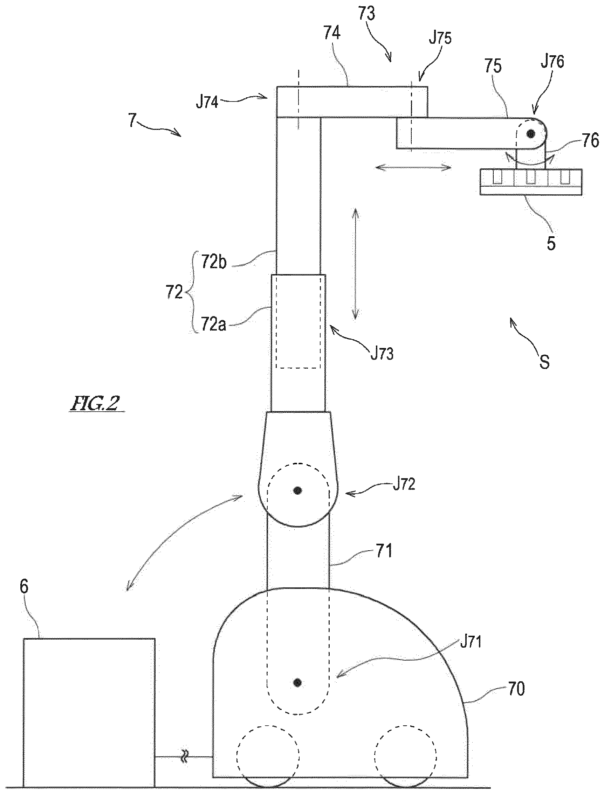

The configuration of the positioner 7 will be described next in detail. Fig. 2 is a side view showing the overall configuration of the positioner 7.

As shown in Fig. 2, the positioner 7 is based on a horizontal multi-joint robot, and includes a base 70 placed on the floor of the operating room, a lifting shaft 72, a swing arm 71 that couples the base 70 to the proximal end portion of the lifting shaft 72, and a horizontal arm 73 coupled to the distal end portion of the lifting shaft 72. The platform 5 is coupled to the distal end portion of the horizontal arm 73.

The base 70 is, for example, a truck with a brake and can be moved to a desired position and made to stand still. The proximal end portion of the swing arm 71 is coupled to the base 70 via a rotary joint J71. By the operation of the rotary joint J71, the swing arm 71 pivots (swings) about the horizontal rotation axis (swing shaft) defined at the base 70. Further, the distal end portion of the swing arm 71 is coupled to the proximal end portion of the lifting shaft 72 via a rotary joint J72. Due to the operation of the rotary joint J72, the swing arm 71 pivots (swings) about the horizontal rotation axis defined at the proximal end portion of the lifting shaft 72.

The lifting shaft 72 extends vertically and is vertically extendable. The lifting shaft 72 according to this embodiment includes a cylindrical member 72a, a hollow shaft member 72b inserted into the cylindrical member 72a so as to be able to advance and retract in the vertical direction, and a translation joint J73 coupling the cylindrical member 72a and the shaft member 72b. Due to the operation of this translation joint J73, the shaft member 72b advances and retracts to and from the cylindrical member 72a in the vertical direction.

The horizontal arm 73 includes a first link 74 and a second link 75 extending horizontally and a wrist link 76 coupled to the distal end portion of the second link 75. The platform 5 is connected to the distal end portion of the wrist link 76.

The proximal end portion of the first link 74 is coupled to the distal end portion of the lifting shaft 72 via a rotary joint J74. The first link 74 and the lifting shaft 72 form a right angle. Due to the operation of the rotary joint J74, the first link 74 pivots about the vertical rotation axis defined at the distal end portion of the lifting shaft 72. The distal end portion of the first link 74 is coupled to the proximal end portion of the second link 75 via a rotary joint J75. Due to the operation of the rotary joint J75, the second link 75 pivots about the vertical rotation axis defined at the distal end portion of the first link 74.

The distal end portion of the second link 75 is coupled to the proximal end portion of the wrist link 76 via a rotary joint J76. Due to the operation of the rotary joint J76, the wrist link 76 pivots about the horizontal rotation axis defined at the distal end portion of the second link 75. The wrist link 76 in the steady state extends vertically and the platform 5 connected to the distal end portion of the wrist link 76 is in a horizontal posture.

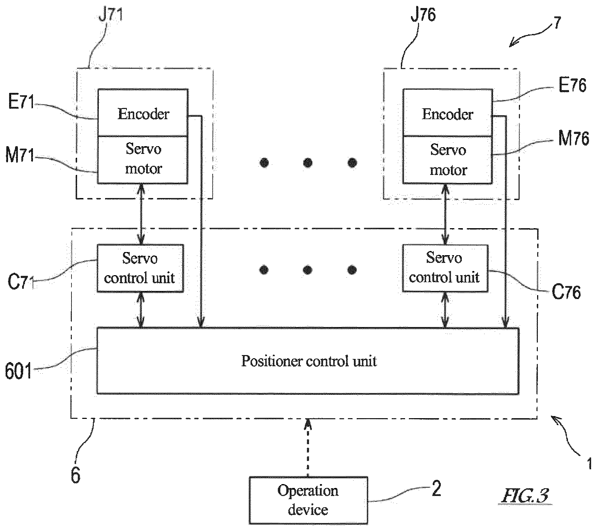

The configuration of the control system of the positioner 7 will be described here. Fig. 3 is a block diagram showing a schematic configuration of the control system of the positioner 7. As shown in Fig. 3, the positioner 7 includes driving servomotors M71 to M76 and encoders E71 to E76 for detecting the rotation angles of the servomotors M71 to M76 in correspondence with the respective joints J71 to J76. Fig. 3 representatively shows a drive system for the rotary joints J71 and J76 among the rotary joints J71 to J76 without showing any drive systems of the remaining joints J73 to J75.

The controller 6 includes a positioner control unit 601 that controls the operation of the positioner 7. Servo control units C71 to C76 are electrically connected to the positioner control unit 601, and the servomotors M71 to M76 are electrically connected to the servo control units C71 to C76 via an amplifier circuit (not shown) and the like.

In the above configuration, a position/posture command for the platform 5 is input to the positioner control unit 601 based on the operation command input to the operation device 2. The positioner control unit 601 generates and outputs a position command value based on the position/posture command and the rotation angle detected by the encoders E71 to E76. The servo control units C71 to C76 that have acquired this position command value generate and output a drive command value (torque command value) based on the rotation angle detected by the encoders E71 to E76 and the position command value. An amplifier circuit that has acquired this drive command value supplies a drive current corresponding to the drive command value to the servomotors M71 to M76. In this manner, the servomotors M71 to M76 are servo-controlled so that the platform 5 reaches the position and posture corresponding to the position/posture command.

As described above, the positioner 7 can change its form in accordance with a position/posture command for the platform 5. As shown in Fig. 2, the basic posture of the positioner 7 is the state in which the swing arm 71 and the lifting shaft 72 extend vertically, the horizontal arm 73 extends horizontally, and the platform 5 connected to the wrist link 76 is in a horizontal posture.

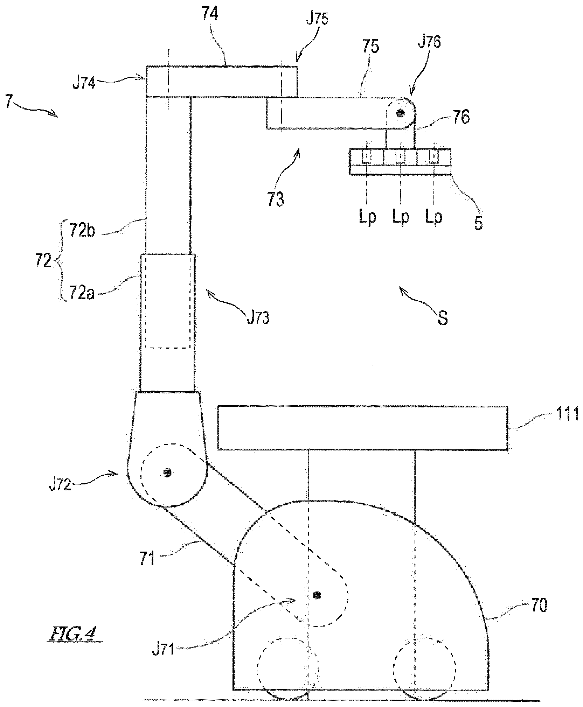

As shown in Fig. 4, when the swing arm 71 in the basic posture is tilted from the vertical position, the positioner control unit 601 operates the rotary joint J71 to tilt the swing arm 71 from the vertical position and rotates the rotary joint J72 to maintain the vertical posture of the lifting shaft 72. In this way, regardless of the tilt of the swing arm 71 from the vertical position, the vertical position of the lifting shaft 72 and the horizontal position of the horizontal arm 73 are maintained.

When the swing arm 71 is tilted from the vertical position as described above, the positioner 7 takes a C shape as a whole. This allows the positioner 7 to take a form in which the base 70 is positioned below the operating table 111, the lifting shaft 72 is positioned on a side of the operating table 111, and the horizontal arm 73 is positioned above the operating table 111. In this way, by accommodating the base 70 below the operating table 111, it is possible to secure the traffic line of an assistant who helps surgery around the operating table 111 during surgery.

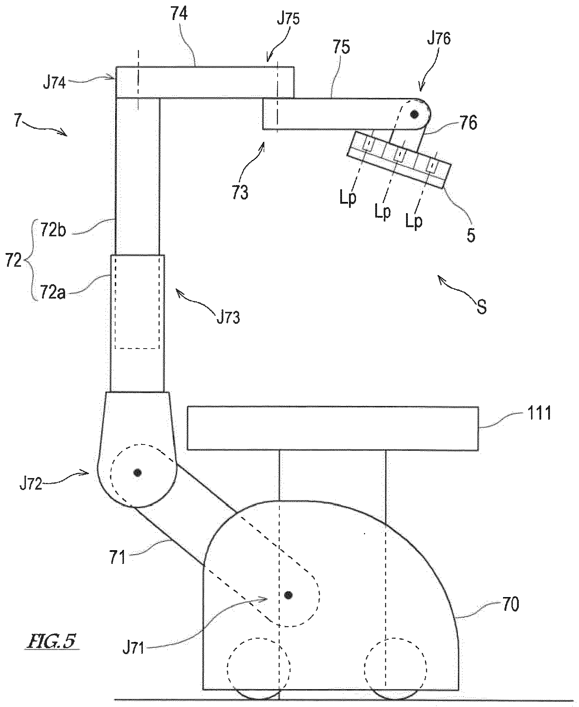

As shown in Fig. 5, when the rotary joint J76 is driven so as to make the wrist link 76 tilt from the vertical position, the platform 5 tilts from the horizontal position. When the platform 5 tilts from the horizontal position, basic axes (pivotal axes) Lp of the arms 3 attached to the platform 5 all tilt from the vertical position at the same time. As a result, the angular range in the reference direction D defined for each instrument 42 is expanded to allow the instrument 42 to be inserted into the patient P while greatly tilting from the vertical position. In this manner, it is possible to appropriately adjust the insertion direction of the instrument 42 with respect to the patient P in accordance with the supine position of the patient P and its surgical position.

Although a preferred embodiment of the positioner 7 has been described above, the configuration of the positioner 7 described above can be changed, for example, as follows.

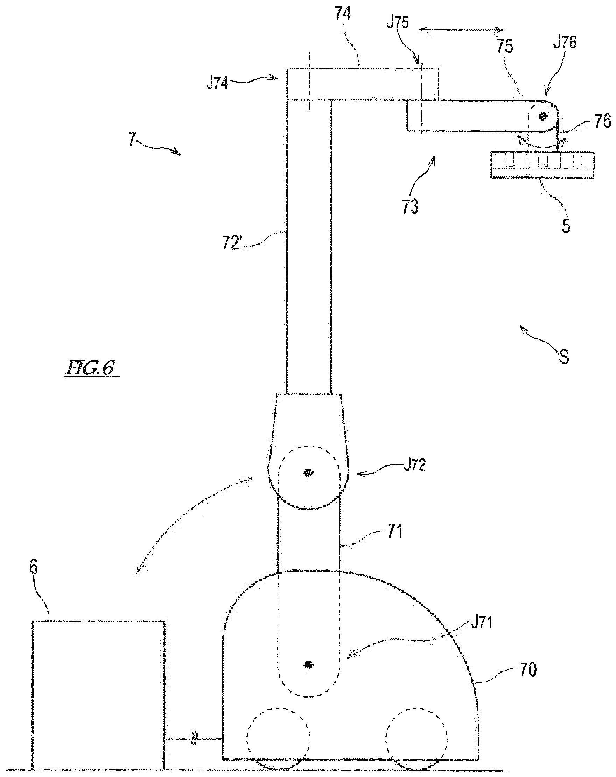

For example, in the positioner 7 according to the above embodiment, the lifting shaft 72 extends and contracts in the vertical direction, but as shown in Fig. 6, a columnar member 72' which does not extend and contract may be used instead of the lifting shaft 72. In this modification, the translation joint J73 is omitted. The positioner 7 according to the above embodiment is configured such that the height position of the platform 5 is adjusted mainly by the extension and contraction of the lifting shaft 72. In contrast to this, in the above modification, the height position of the platform 5 is adjusted mainly by the tilting of the swing arm 71 from the vertical position.

### [Configuration Example of Arm]

The configuration of each arm 3 will be described in detail here. Fig. 7 shows a schematic configuration of one of the plurality of arms 3 of the patient-side system 1. As shown in Fig. 7, each arm 3 includes an arm body 30 and a translation arm 35 coupled to the distal end portion of the arm body 30, and is configured to move the distal end portion in a three-dimensional space with respect to the proximal end portion. In this embodiment, each of the plurality of arms 3 of the patient-side system 1 has the same or similar configuration, but at least one of the plurality of arms 3 may have a different configuration.

When the arm 3 is the instrument arm 3B, a holder (instrument holder) 36 for holding the instrument 42 is provided at the distal end of the translation arm 35. The instrument 42 is detachably held by the holder 36. The shaft 43 of the instrument 42 held by the holder 36 extends in parallel with the reference direction D.

When the arm 3 is the camera arm 3A, like the instrument arm 3B, the holder 36 is provided at the distal end portion of the translation arm 35, and the endoscope assembly 41 is detachably held by the holder 36. In this case, the holder 36 provided for the camera arm 3A may have a different form from that of the holder 36 provided for the instrument arm 3B. Alternatively, because the endoscope assembly 41 is rarely changed during surgery, the endoscope assembly 41 may be fixed to the camera arm 3A.

The arm 3 is detachable with respect to the platform 5 (that is, easy to attach and detach), and has water resistance, heat resistance, and chemical resistance for a cleaning process and a sterilization process. There are various methods for sterilizing the arms 3. For example, a high pressure steam sterilization method, an EOG sterilization method, and a chemical sterilization method with a disinfectant may be selectively used. In the high-pressure steam sterilization method, each arm 3 is sealed in a high-pressure container such as an autoclave and exposed to saturated steam at a predetermined pressure for a predetermined time (for example, for 30 min at 115°C, for 20 min at 121°C , or for 15 min at 126°C). In the EOG sterilization method, each arm 3 is sealed in a container, and 450 mg/L to 1000 mg/L of ethylene oxide gas is circulated in the container. In the chemical sterilization method, for example, each arm 3 is immersed in a disinfectant such as glutaral.

### [Configuration Example of arm body]

The arm body 30 includes a base 80 detachably attached to the platform 5 and first to sixth links 81 to 86 sequentially coupled to each other from the base 80 to the distal end portion. More specifically, the proximal end portion of the first link 81 is coupled to the distal end portion of the base 80 via a torsional joint J31. The proximal end portion of the second link 82 is coupled to the distal end portion of the first link 81 via a torsional joint J32. The proximal end portion of the third link 83 is coupled to the distal end portion of the second link 82 via a bending joint J33. The proximal end portion of the fourth link 84 is coupled to the distal end portion of the third link 83 via a torsional joint J34. The proximal end portion of the fifth link 85 is coupled to the distal end portion of the fourth link 84 via a bending joint J35. The proximal end portion of the sixth link 86 is coupled to the distal end portion of the fifth link 85 via a torsional joint J36. The proximal end portion of the translation arm 35 is coupled to the distal end portion of the sixth link 86.

The outer shell of the arm body 30 is mainly made of a member having heat resistance and chemical resistance such as stainless steel. In addition, a seal (not shown) for providing water resistance is provided for the coupling portion between the links. This seal has heat resistance corresponding to the high pressure steam sterilization method and chemical resistance against a disinfectant. In the coupling portion between the links, the end portion of the other link is inserted into the end portion of one link to be coupled, and a seal is disposed so as to fill the space between the end portions of these links, thereby hiding the seal from the outside. This prevents infiltration of water, chemical liquid, and steam from between the seal and the link.

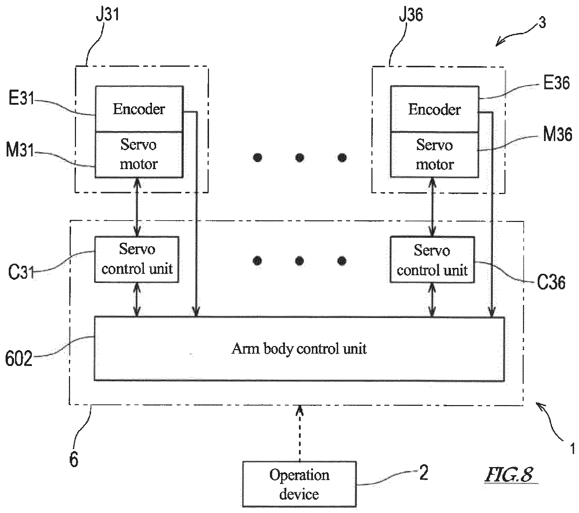

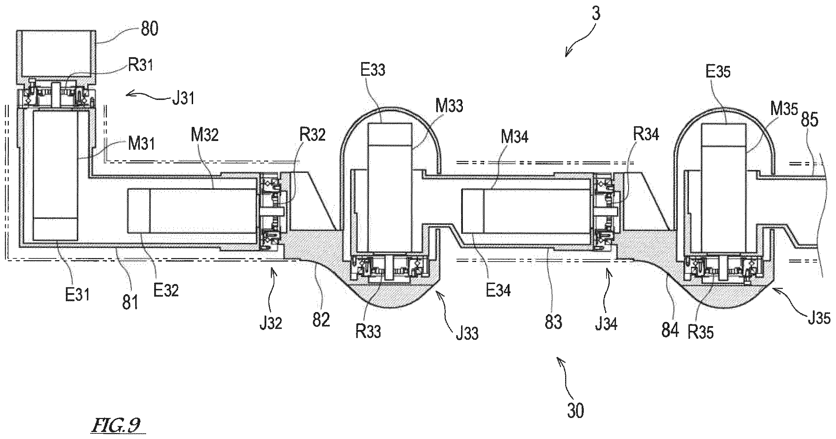

The configuration of a drive system and a control system for the arm body 30 will be described with reference to Figs. 8 and 9. Fig. 8 is a block diagram showing a schematic configuration of the control system for the arm body 30. Fig. 9 is a schematic sectional view of the arm body 30, showing the layout of the drive system for the arm body 30.

In accordance with the respective joints J31 to J36, the arm body 30 having the above configuration is provided with driving servomotors M31 to M36, encoders E31 to E36 for detecting the rotation angles of the servomotors M31 to M36, and speed reducers R31 to R36 for increasing torques by reducing the outputs of the servomotors M31 to M36. Fig. 8 representatively shows a control system for the torsional joint J31 and the torsional joint J36 among the joints J31 to J36 without showing control systems for the remaining joints J33 to J35. The encoders E31 to E36 each are provided as an example of a rotational position detector for detecting the rotational position (rotational angle) of each of the servomotors M31 to M3. Instead of the encoders E31 to E36, a rotational position detector such as a resolver may be used. In addition, each of the above-described elements of the drive system for the arm body 30 and wirings and control unit for the elements are made of a high-temperature-resistant material and are provided with heat resistance for a sterilization process.

At the torsional joint J31 coupling the base 80 and the first link 81, the servomotor M31 is provided at the proximal end portion of the first link 81, and the speed reducer R31 is provided at the distal end portion of the base 80. The speed reducer R31 according to this embodiment is of a unit type including a gear that reduces the rotational speed based on the input power and an output gear that receives an output from the gear. The servomotor M31 is disposed such that its output shaft is parallel to the rotation axis of the torsional joint J31. The encoder E31 is attached to the servomotor M31. The output from the servomotor M31 is input to the speed reducer R31. Because the output gear of the speed reducer R31 is fixed to the first link 81, the first link 81 is rotated with respect to the base 80 by the output from the speed reducer R31.

At the torsional joint J32 coupling the first link 81 and the second link 82, the servomotor M32 is provided at the distal end portion of the first link 81, and the reduction gear R32 is provided at the proximal end portion of the second link 82. The servomotor M32 is disposed such that its output shaft is parallel to the rotation axis of the torsional joint J32. The encoder E32 is attached to the servomotor M32.

At the bending joint J33 coupling the second link 82 and the third link 83, the speed reducer R33 is provided at the distal end portion of the second link 82, and the servomotor M33 is provided at the proximal end portion of the third link 83. The servomotor M33 is disposed such that its output shaft is parallel to the rotation axis of the bending joint J33. The encoder E33 is attached to the servomotor M33.

In the manner as described above, the servomotors M34 to M36, the encoders E34 to E36, and the speed reducers R34 to R36 are also arranged at the remaining joints J34 to J36.

The servomotors M31 to M36 are small in output (for example, on the order of 80 W), lightweight, and small in size. In addition, the speed reducers R31 to R36 each adopt a speed reducer having a flat shape with a small axial dimension and capable of obtaining a high torque at a high reduction ratio (for example, 100 or more). A high-speed operation like that of a general industrial manipulator is not required for each arm 3 of the patient-side system 1, and hence a servomotor with a large output is not required. Accordingly, by using a combination of the servomotors M31 to M36 having relatively small outputs and the speed reducers R31 to R36 having relatively high reduction ratios, it is possible to achieve reductions in weight and size of the arms 3 while securing necessary torques.

In addition, in a general industrial manipulator, an output from a servomotor is transmitted to the output gear, the speed reducer, and the load in the order named. In each arm 3 according to this embodiment, an output of the servomotor is transmitted to the speed reducer, the output gear, and the load in the order named. By thus laying out the speed reducer on the input side with respect to the output gear, weight reduction and miniaturization of the arm 3 are achieved.

The controller 6 includes an arm body control unit 602 that controls the operation of the arm body 30. Servo control units C31 to C36 are electrically connected to the arm body control unit 602, and the servomotors M31 to M36 are electrically connected to a servo control unit 79 via an amplifier circuit (not shown) and the like.

In the above configuration, a position/posture command for the distal end portion of the arm body 30 is input to the arm body control unit 602 based on the operation command input to the operation device 2. The arm body control unit 602 generates and outputs a position command value based on the position/posture command and the rotation angle detected by the encoders E31 to E36. The servo control units C31 to C36 that have acquired this position command value generate and output a drive command value (torque command value) based on the rotation angle detected by each of the encoders E31 to E36 and the position command value. An amplifier circuit that has acquired this drive command value supplies a drive current corresponding to the drive command value to the servomotors M31 to M36. In this manner, the servomotors M31 to M36 are servo-controlled so that the distal end of the arm body 30 reaches the position and posture corresponding to the position/posture command.

### [Coupling Structure between Arm and Platform]

A coupling structure between the platform 5 and each arm 3 will be described here.

The base 80 of each arm 3 is detachable from the platform 5. In other words, it is easy to detach and attach the whole arm 3 to and from the patient-side system 1. In this embodiment, the four arms 3 are detachable from the platform 5, but at least one of the arms 3 of the patient-side system 1 may be detachable from the platform 5.

The arm 3 detached from the patient-side system 1 is subjected to a cleaning process and a sterilization process and then reused within a limited number of times. In this way, the arm 3 can be replaced with a clean one that is sterilized for every operation. Accordingly, the arm 3 may not be covered with a sterile drape as in the conventional art, but may be exposed to a sterile field.

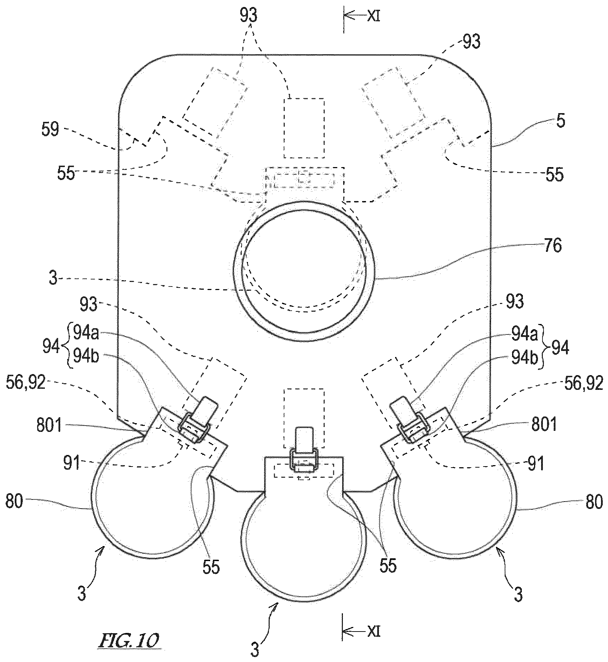

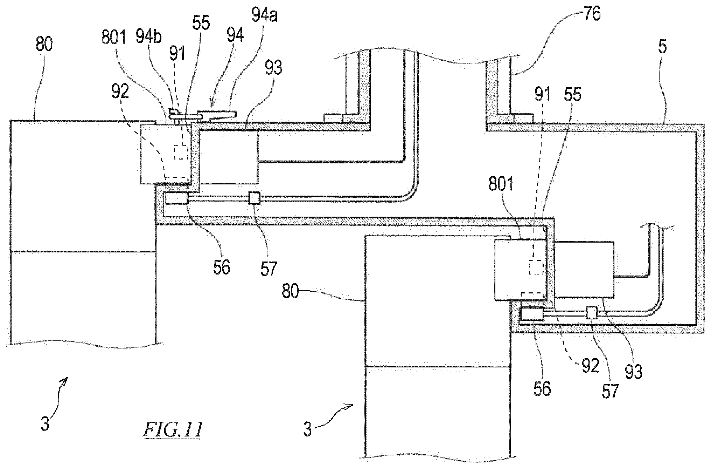

Fig. 10 is a plan view showing a coupling structure between the platform 5 and each arm 3. Fig. 11 is a sectional view taken along a line XI-XI in Fig. 10. As shown in Figs. 10 and 11, the proximal end portion of the base 80 of each arm 3 has a cylindrical shape, and at least one interface unit (to be sometimes referred to as an "I/F unit 801" hereinafter) on the circumference of the proximal end portion or on the proximal end face. The I/F unit 801 according to this embodiment is a protrusion formed on the outer circumferential surface of the base 80, but the form of the I/F unit 801 is not limited to this.

In the I/F unit 801, an IC tag 91 for attaching identification information and the like to the arm 3 is embedded. The IC tag 91 includes an IC chip and an antenna, and the IC chip includes a microcomputer, an EEPROM, a RAM (neither of which is shown). The IC tag 91 stores the individual identification information, the model number, the usage count, and the like of the arm 3.

In the I/F unit 801 is provided with one or more connectors 92. The one or more connectors 92 include a connector for electric wires for supplying electricity to the arm 3 and a connector for communication wiring for transmitting/receiving signals to/from the arm 3.

On the other hand, the platform 5 is provided with an attachment port 55 to which the I/F unit 801 of the base 80 is connected. The attachment port 55 (an example of the manipulator arm attachment portion) according to this embodiment is a concave portion into which the protruding I/F unit 801 can be fitted, but the form of the attachment port 55 is not limited to this.

In this embodiment, because the four arms 3 are detachably attached to the platform 5, at least four attachment ports 55 are provided on the platform 5. The platform 5 has a hexagonal shape obtained by chamfering two adjacent corners of a quadrangle in plan view, and three continuous side surfaces of the hexagonal shape have components extending in the same direction. One attachment port 55 is provided for each of these three side surfaces. A part of the lower part of the platform 5 is cut out so as to form a wall 59 facing laterally, and three lower attachment ports 55 are provided to the wall 59 in the same manner as the three upper attachment ports 55. In this embodiment, one of the three lower attachment ports 55 is used, and the remaining two attachment ports are empty. As described above, the platform 5 is provided with the plurality of attachment ports 55, and it is possible to select the attachment port 55 to be used for each operation.

As described above, the I/F unit 801 provided on the base 80 of the arm 3 and the attachment port 55 provided in the platform 5 constitute a coupling mechanism for coupling the arm 3 and the platform 5. The base 80 (that is, the arm 3) is attached to the platform 5 by fitting the I/F unit 801 of the base 80 into the attachment port 55 of the platform 5.

The attachment port 55 of the platform 5 is provided with a socket 56 at a position corresponding to the connector 92 provided for the I/F unit 801. As the I/F unit 801 and the attachment port 55 are coupled, the connector 92 and the socket 56 are automatically connected. An electric wires and/or communication wirings are connected to the socket 56 via inner spaces of hollow elements (shafts, links, and the like) constituting the platform 5 and the positioner 7. Although the connector 92 is exposed on the surface of the arm 3 and can be brought into contact with the socket 56, the connector 92 may be embedded in the vicinity of the surface of the arm 3, and the socket 56 and the connector 92 may be electrically connected to each other contactlessly by electromagnetic induction. In addition, the socket 56 may be provided in the I/F unit 801 and the connector 92 may be provided in the attachment port 55.

The platform 5 is provided with a reader/writer 93 for reading and writing (storing) information with respect to the IC tag 91 embedded in the arm 3. The reader/writer 93 is provided in correspondence with each attachment port 55 of the platform 5, and outputs information read from the IC tag 91 to the controller 6 (to be described later). The reader/writer 93 may individually read the IC tag 91 of each arm 3 attached to the platform 5, or may simultaneously read the IC tags 91 of all the arms 3 attached to the platform 5.

The platform 5 and the base 80 are provided with one or more pairs of attachment lock mechanisms 94 that can implement attachment locking (holding) and attachment unlocking (holding release) of the arm 3 attached to the platform 5 so as to prevent the base 80 from falling off from the platform 5. Note that attachment locking means fixing the I/F unit 801 of the arm 3 attached to the attachment port 55 of the platform 5 to the attachment port 55, and attachment unlocking means releasing the fixation.

The attachment lock mechanism 94 is implemented by cooperation between a support body side engagement portion 94a provided on or near the attachment port 55 of the platform 5 and an arm side engagement portion 94b provided on or near the I/F unit 801 of the arm 3. One of the support body side engagement portion 94a and the arm side engagement portion 94b is engaged with the other, and the other way around. In the attachment lock mechanism 94, the I/F unit 801 is locked while being attached to the attachment port 55 by the engagement between the support body side engagement portion 94a and the arm side engagement portion 94b, and the I/F unit 801 can be separated from the attachment port 55 by disengagement between the support body side engagement portion 94a and the arm side engagement portion 94b.

The attachment lock mechanism 94 as described above is selected from the group consisting of, for example, a pair of a protrusion provided on one of the platform 5 and the base 80 and a latch lever provided on the other, a pair of a recess provided in one of the platform 5 and the base 80 and an engagement pawl provided on the other, and a pair of a recess provided in one of the platform 5 and the base 80 and a ball plunger provided on the other. Alternatively, the attachment lock mechanism 94 may be another known attachment lock mechanism. However, it is desirable that the attachment lock mechanism 94 allows locking/unlocking by a one-touch operation instead of using a tool for locking/unlocking such as bolts, nuts, and the like.

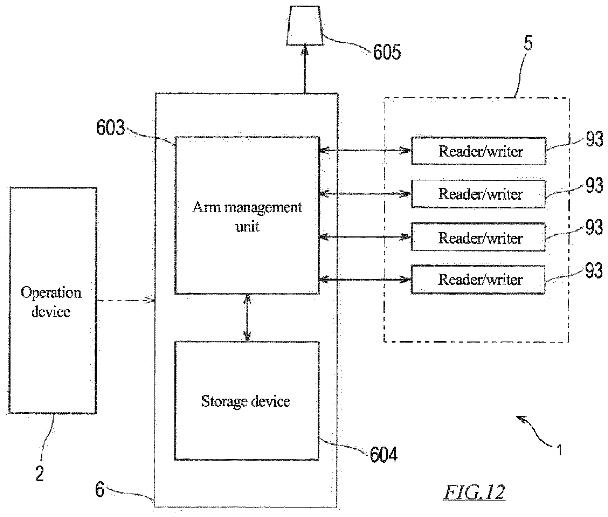

Fig. 12 is a block diagram showing a configuration for managing the arm 3 attached to the platform 5. As shown in Fig. 12, the controller 6 includes an arm management unit (management device) 603 for managing the arm 3 attached to the platform 5. The reader/writer 93 is electrically connected to the arm management unit 603.

The arm management unit 603 detects connection between the connector 92 and the socket 56 based on power supply from the platform 5 to the arm 3. Power supply from the platform 5 to the arm 3 can be detected based on, for example, a detection signal from a current detection sensor (detection sensor 57) provided on an electric wire or communication wiring up to the socket 56. Connection between the connector 92 and the socket 56 indicates that the I/F unit 801 is normally attached to the attachment port 55. In other words, the presence/absence of the arm 3 attached to the attachment port 55 can be detected based on the presence/absence of power supply from the platform 5 to the arm 3. In this manner, the arm management unit 603 can detect that the arm 3 is attached to each attachment port 55 of the platform 5.

However, in order to detect the presence/absence of the arm 3 attached to the attachment port 55, a contact type or non-contact type object detection sensor (not shown) may be provided on the platform 5. In this case, the arm management unit 603 detects that the arm 3 is attached to the attachment port 55 based on a detection signal from this detection sensor.

Upon detecting attachment of the arm 3 to the attachment port 55, the arm management unit 603 causes the reader/writer 93 to perform a read operation, and acquires individual identification information, model number information (type), usage count information, and the like of each arm 3 connected to the platform 5, based on the information read by the reader/writer 93 from the IC tag 91. The arm management unit 603 temporarily stores each acquired information in association with the attachment position information (that is, the attachment port 55) on the platform 5 to which the arm 3 is attached. Each of the plurality of attachment ports 55 is identified.

Surgical information is previously input to the controller 6 via the operation device 2 and set (stored). This surgical information includes a combination of a plurality of arms 3 used in surgery.

The arm management unit 603 determines whether the combination of the individual identification information included in the information acquired from the reader/writer 93 corresponds to the combination set as the surgical information. If the combination does not correspond to the one set as surgical information, the arm management unit 603 outputs a warning via an alarm unit 605 connected to the controller 6. It is to be noted that the alarm unit 605 warns the operator O by one or more of light, sound, and image. In this way, the arm management unit 603 manages the arms 3 attached to the platform 5 so as to attach the appropriate arms 3.

The surgical information may include information on the combination of the individual identification information of the arm 3 used in surgery and the attachment position of the platform 5 to which the arm 3 is to be attached (i.e., the attachment port 55).

In this case, the arm management unit 603 determines whether the combination of the individual identification information included in the information acquired from the reader/writer 93 and the attachment position information (that is, the attachment port 55) on the platform 5 stored in association with the individual identification information corresponds to the information set as the surgical information. If the combination does not correspond to the one set as surgical information, the arm management unit 603 outputs a warning via an alarm unit 605 connected to the controller 6. In this way, the arm management unit 603 manages the arms 3 attached to the platform 5 so as to attach each arm 3 to an appropriate position on the platform 5 and attach the appropriate arms 3 to the respective attachment ports 55.

The surgical information may also include information on the combination of the model number information of the arm 3 used in surgery and the attachment position on the platform 5 on which the arm 3 is to be attached (i.e., the attachment port 55).

In this case, the arm management unit 603 may be configured to determine whether the combination of the model number information included in the information acquired from the reader/writer 93 and the attachment position (attachment port 55) associated with the model number information corresponds to the information set as the surgical information and, upon determining that the combination does not correspond to the information set as the surgical information, output a warning via the alarm unit 605 connected to the controller 6.

The types of the arm 3 (the camera arm 3A and the instrument arm 3B), the structure (the length of the link, the degree of freedom, and the like) and the like are different depending on the model number. Although the model number information is stored in the IC tag 91 in the above description, a storage device 604 may include a model number storage unit in which the model number information is stored in association with the individual identification information, and the arm management unit 603 may read out, based on the individual identification information, the corresponding model number information from the model number storage unit. This model number information may be used in place of the model number information read out from the IC tag 91 in the above processing.

The storage device 604 of the controller 6 includes a usage limit number storage unit that stores the usage limit number associated with individual identification information. Based on the individual identification information acquired from the IC tag 91, the arm management unit 603 reads the usage limit number corresponding to the individual identification information from the storage device 604, and compares the usage limit number with the acquired usage count information. If the usage count information exceeds the usage limit number, the arm management unit 603 outputs a warning via the alarm unit 605 connected to the controller 6. In this way, the arm management unit 603 manages the usage count of the arm so that the arm 3 will not be used beyond its usage limit number. The arm 3 is a consumable item, and the arm 3 which has been used up to the usage limit number is discarded.

The arm management unit 603 causes the reader/writer 93 to operate so as to write new usage count information obtained by adding 1 to the usage count information acquired from the IC tag 91 in the IC tag 91. As a result, the IC tag 91 of the arm 3 holds information on the usage count of the arm itself. This makes it possible to share the arms 3 with another patient-side system 1.

In the above description, the arm 3 itself holds the usage count information of the arm 3, but the usage count information of the arm 3 may be stored in the storage device 604 of the controller 6. In this case, instead of the reader/writer 93, a reader having only a reading function may be used. Based on the individual identification information read by the reader from the IC tag 91, the arm management unit 603 may then read the corresponding usage count information from the storage device 604 and use it for the above processing.

Although a preferred embodiment of the coupling structure between the platform 5 and each arm 3 has been described above, the coupling structure between the platform 5 and each arm 3 can be changed, for example, as follows.

For example, in the above embodiment, the platform 5 is provided with six attachment ports 55, and the arms 3 are connected to four of these attachment ports 55. In this way, there may be an empty attachment port 55 among the plurality of attachment ports 55. In addition, on the platform 5, five or more attachment ports 55 may be provided, and the attachment port 55 at an appropriate position corresponding to the contents of surgery may be selectively used.

For example, in the above-described embodiment, each arm 3 is provided with the IC tag 91 in order to hold the information in the arm 3 itself. However, the IC tag 91 is an example of an information holding unit provided on the arm 3, and another information holding unit may be used in place of or in addition to the IC tag 91. For example, a bar code may be provided on the arm 3 and a bar code reader may be provided on the platform 5. In addition, for example, a shape symbol representing unevenness or the like may be provided on the arm 3 and a reader for reading the shape symbol may be provided on the platform 5.

For example, in the above embodiment, the connector 92 is provided for the I/F unit 801 of the base 80 of each arm 3 and the socket 56 is provided in the attachment port 55 of the platform 5, but the connector 92 and the socket 56 may be omitted. In this case, wires for power supply and/or wiring for communication are connected to the arm 3 other than the I/F unit 801.

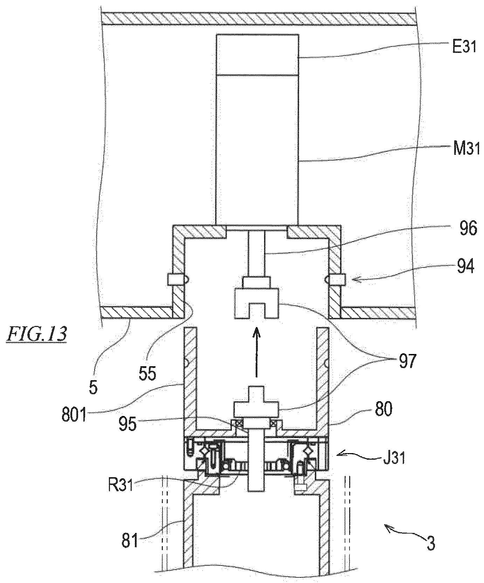

For example, in the above-described embodiment, the overall drive system of each arm 3 (particularly, the arm body 30) is mounted on itself, but part of the drive system of the arm body 30 may be provided on the platform 5. For example, as shown in Fig. 13, a servomotor M31 for driving the torsional joint J31 coupling the base 80 and the first link 81 may be provided in the platform 5 .

In the example shown in Fig. 13, the servomotor M31 is mounted in the platform 5, and the attachment port 55 in the form of a female coupler is provided around an output shaft 96 of the servomotor M31. On the other hand, the output portion of the speed reducer R31 is fixed to the proximal end portion of the first link 81 of the arm 3, and the proximal end portion of the base 80 serves as the I/F unit 801 in the form of a male coupler corresponding to the attachment port 55. An input shaft 95 for inputting power to the speed reducer R31 is provided in the base 80. The input shaft 95 transmits power to the power system of the arm 3. A shaft coupling 97 such as an Oldham coupling or the like is provided between the output shaft 96 of the servomotor M31 and the input shaft 95 to the speed reducer R31. In the above configuration, when the I/F unit 801 of the arm 3 is inserted into the attachment port 55 of the platform 5, the platform 5 and the arm 3 are coupled and the output shaft 96 of the servomotor M31 is coupled to the input shaft 95 to the speed reducer R31 so as allow transmission of power.

### [Configuration Example of Translation Arm]

As shown in Fig. 7, the translation arm 35 is a mechanism that translates the holder 36 attached to the distal end portion of the translation arm 35 in the reference direction D to translate the instrument 42 attached to the holder 36 in the extending direction of the shaft 43.

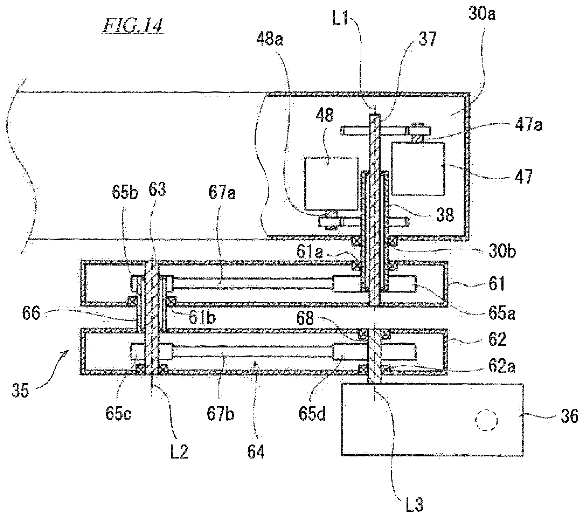

Fig. 14 is a partially cutaway view showing a configuration example of the distal end portion of the arm body 30 and the translation arm 35. As shown in Figs. 7 and 14, the translation arm 35 includes a proximal end side link 61, a distal end side link 62, a first coupling shaft 63 coupling the proximal end side link 61 and the distal end side link 62, a second coupling shaft 66, and an interlocking mechanism 64. In addition, a pivot shaft 68 is provided at the distal end portion of the translation arm 35, that is, the distal end portion of the distal end side link 62.

As shown in Fig. 14, the drive source of the translation arm 35 is provided on the link at the distal end of the arm body 30, that is, on the sixth link 86. More specifically, the distal end portion of the sixth link 86 is provided with a first translation arm drive shaft 37, a second translation arm drive shaft 38, a translation arm drive unit 47 for causing the first translation arm drive shaft 37 to pivot, and a translation arm rotation drive unit 48 for causing the second translation arm drive shaft 38 to pivot.

The first translation arm drive shaft 37 and the second translation arm drive shaft 38 are attached such that their proximal end portions are attached to the distal end portion of the arm body 30 so as to be pivotal about a first axis L1 orthogonal to the reference direction D. The proximal end portion of the second translation arm drive shaft 38 is held by the arm body 30 via a seal bearing 30b having a seal for blocking between the internal and external environments of the arm body 30. The second translation arm drive shaft 38 is formed in a hollow cylindrical shape, and the first translation arm drive shaft 37 is nested inside the second translation arm drive shaft 38. Accordingly, the first translation arm drive shaft 37 and the second translation arm drive shaft 38 are configured to pivot about the same axis. In this embodiment, the first axis L1 is configured to extend in a tangential direction of a circle centered on a swivel axis Lp (see Fig. 7) of the arm body 30. That is, the first axis L1 extends in the depth direction in Fig. 7. The first translation arm drive shaft 37 and the second translation arm drive shaft 38 protrude from the distal end portion of the arm body 30 and are coupled to the proximal end side link 61 of the translation arm 35. That is, the arm body 30 and the translation arm 35 are coupled via the first translation arm drive shaft 37 and the second translation arm drive shaft 38. The first translation arm drive shaft 37 is a drive shaft that causes the translation arm 35 to operate by differential from the second translation arm drive shaft 38. The second translation arm drive shaft 38 is a drive shaft that causes the translation arm 35 to pivot about the first axis L1 by differential from the first translation arm drive shaft 37.

The first translation arm drive shaft 37 and the second translation arm drive shaft 38 are configured to independently pivot using separate drive units. That is, the first translation arm drive shaft 37 is coupled to an output shaft 47a of the translation arm drive unit 47 and rotates by the rotation of the output shaft 47a of the translation arm drive unit 47. In addition, the second translation arm drive shaft 38 is coupled to an output shaft 48a of the translation arm rotation drive unit 48 and rotates by the rotation of the output shaft 48a of the translation arm rotation drive unit 48. These drive units are, for example, servomotors. Therefore, the angular positions of the first translation arm drive shaft 37 and the second translation arm drive shaft 38 around the first axis L1 can be controlled independently of each other.

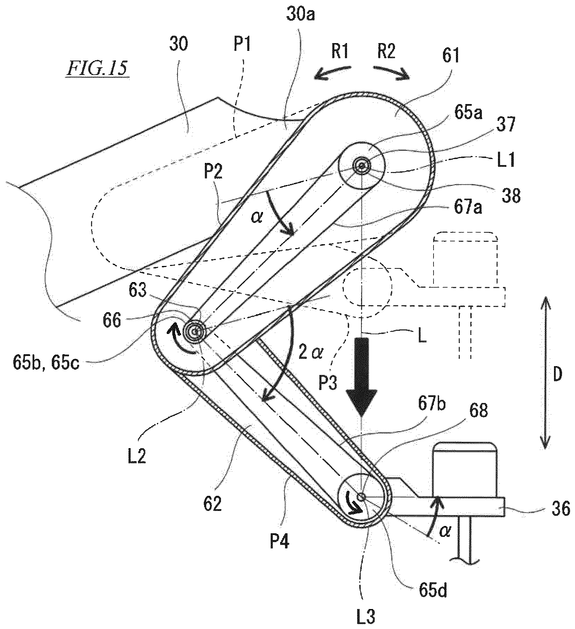

The proximal end portion of the proximal end side link 61 is continuous with a distal end portion 30a of the arm body 30 so as to be pivotal about the first axis L1. It should be noted that "continuous" means not only that two objects are directly connected, but also that two objects are indirectly connected to each other with another object being interposed between them. The proximal end side link 61 is configured to pivot within a range including a retraction angular position P1 (see Fig. 15) and an advancement angular position P2 (see Fig. 15) where the distal end portion of the proximal end side link 61 is located further from the first axis L1 in the reference direction D than the retraction angular position P1. The proximal end side link 61 is hollow. The distal end portions of the first translation arm drive shaft 37 and the second translation arm drive shaft 38 extend through the proximal end portion of the proximal end side link 61, and the second translation arm drive shaft 38 positioned on the outer side is held on the proximal end side link 61 via a seal bearing 61a having a seal for blocking between the inner and outer spaces. The distal end portion of the first translation arm drive shaft 37 is fixed to the proximal end side link 61. Accordingly, the rotation of the output shaft 47a of the translation arm drive unit 47 rotates the first translation arm drive shaft 37, and the rotation of the first translation arm drive shaft 37 causes the proximal end side link 61 to pivot about the first axis L1. In this manner, the proximal end side link 61 is attached to the distal end portion 30a of the arm body 30.

The proximal end portion of the distal end side link 62 is continuous with the distal end portion of the proximal end side link 61 so as to be pivotal about a second axis L2 extending in parallel with the axis L1. The distal end side link 62 is configured to pivot within a range including a closing angular position P3 (see Fig. 15) and an opening angular position P4 where the angle formed with the proximal end side link 61 is larger than that formed with the closing angular position P3. In this way, the proximal end side link 61 and the distal end side link 62 are configured to assume a bent posture in an L shape. The distal end side link 62 is hollow. The proximal end portion of the first coupling shaft 63 is fixed to the distal end portion of the proximal end side link 61 and the distal end portion is attached to the proximal end portion of the distal end side link 62 so as to be pivot about the second axis L2. The proximal end portion of the second coupling shaft 66 is attached to the proximal end side link 61 via the seal bearing 61b having a seal for blocking between the inner and outer spaces of the proximal end side link 61 so as to be pivotal about the second axis L2, and the distal end portion is fixed to the proximal end portion of the distal end side link 62. The second coupling shaft 66 is formed in a hollow cylindrical shape, and the first coupling shaft 63 is nested inside the second coupling shaft 66. Accordingly, the first coupling shaft 63 and the second coupling shaft 66 are configured to pivot about the same axis. The translation arm 35 is configured such that an imaginary straight line L (see Fig. 15) connecting the proximal end portion of the proximal end side link 61 and the distal end portion of the distal end side link 62 extends in the reference direction D. In this manner, the proximal end portion of the distal end side link 62 is attached to the distal end portion of the proximal end side link 61.

The proximal end portion of the pivot shaft 68 is attached to the distal end portion of the distal end side link 62 so as to be pivotal about a third axis L3 extending in parallel with the first axis L1 and the second axis L2, and the distal end portion is fixed to the holder 36. The pivot shaft 68 is held at the distal end portion of the distal end side link 62 via a seal bearing 62a having a seal for blocking between the inner and outer spaces of the distal end side link 62. In this embodiment, the translation arm 35 is disposed so as to be located on the side from the proximal end portion toward the distal end portion of the shaft 43 of the instrument 42 held by the holder 36 in the reference direction D from the distal end portion 30a of the arm body 30. The distance between the axis (the third axis L3) of the pivot shaft 68 and the axis (the second axis L2) of the first coupling shaft 63 and the second coupling shaft 66 is equal to the distance between the axis (second axis L2) of the first translation arm drive shaft 37 and the second translation arm drive shaft 38 and the axis (first axis L1) of the first coupling shaft 63 and the second coupling shaft 66.

The interlocking mechanism 64 is a mechanism for causing the distal end side link 62 to pivot from the closing angular position P3 (see Fig. 15) toward the opening angular position P4 (see Fig. 15) in conjunction with the pivoting operation of the proximal end side link 61 from the retraction angular position P1 (see Fig. 15) toward the advancement angular position P2 (see Fig. 15) and also causing the distal end side link 62 to pivot from the opening angular position P4 (see Fig. 15) toward the closing angular position P3 (see Fig. 15) in conjunction with the pivoting operation of the proximal end side link 61 from the advancement angular position P2 (see Fig. 15) toward the retraction angular position P1 (see Fig. 15). That is, the distal end side link 62 is configured to pivot as the driving force of the translation arm drive unit 47 is transmitted by the interlocking mechanism 64. In addition, the interlocking mechanism 64 is configured to cause the distal end portion of the distal end side link 62 to linearly move in the reference direction D. A mechanism using a timing belt will be described below as an example of the interlocking mechanism 64.

In this embodiment, the interlocking mechanism 64 includes two pairs of pulleys (a first pulley 65a, a second pulley 65b, a third pulley 65c, and a fourth pulley 65d) and two annular timing belts (first belt 67a and second belt 67b) wound around each pair of pulleys.

The first pulley 65a is fixed to the distal end portion of the second translation arm drive shaft 38, and is located at the proximal end side of the inner space of the proximal end side link 61. Accordingly, the first pulley 65a is configured to pivot about the first axis L1.

The second pulley 65b forms a pair with the first pulley 65a and is fixed to the proximal end portion of the second coupling shaft 66, and is located at the distal end side of the inner space of the proximal end side link 61. Accordingly, the second pulley 65b is configured to pivot about the second axis L2. The first belt 67a is wound around the first pulley 65a and the second pulley 65b. Accordingly, the second pulley 65b is configured to pivot (rotate) by revolving around the first pulley 65a. The pulley ratio that is the ratio of the diameter of the first pulley 65a to the diameter of the second pulley 65b is 2: 1.

The third pulley 65c is fixed to the distal end portion of the first coupling shaft 63 and is located at the proximal end side of the inner space of the distal end side link 62. Accordingly, the third pulley 65c is configured to pivot about the second axis L2.

The fourth pulley 65d forms a pair with the third pulley 65c and is fixed to the proximal end portion of the pivot shaft 68, and is located at the distal end side of the inner space of the distal end side link 62. Accordingly, the fourth pulley 65d is configured to pivot about the third axis L3. The second belt 67b is wound around the third pulley 65c and the fourth pulley 65d. Accordingly, the fourth pulley 65d is configured to pivot (rotate) by revolving around the third pulley 65c. The pulley ratio that is the ratio of the diameter of the third pulley 65c to the diameter of the fourth pulley 65d is 1: 2. Driving of the translation arm 35 is performed by making the controller 6 control the first translation arm drive shaft 37 and the second translation arm drive shaft 38 based on the operation command input to the operation device 2.

Fig. 15 is a view showing an operation example of the translation arm 35.

An operation example of the translation arm 35 will be described below with reference to Fig. 15.

When the first translation arm drive shaft 37 is differentially operated with respect to the second translation arm drive shaft 38 and is rotated through α° in the R1 direction with respect to the second translation arm drive shaft 38, the proximal end side link 61 pivots through α° from the retraction angular position P1 toward the advancement angular position P2. As a result, the second pulley 65b revolves around the first axis L1. Because the first pulley 65a and the second pulley 65b are coupled by the first belt 67a, the second pulley 65b rotates relative to the first pulley 65a in the direction opposite to the rotation direction of the proximal end side link 61, resulting in a change in phase between the first pulley 65a and the second pulley 65b. The angular displacement is then -2α° based on the pulley ratio. When the second pulley 65b rotates, the second coupling shaft 66 to which the second pulley 65b is fixed rotates, and the distal end side link 62 fixed to the second coupling shaft 66 rotates through -2α° from the closing angular position P3 toward the opening angular position P4. As a result, the distal end portion of the distal end side link 62 moves on an imaginary straight line L connecting the proximal end portion of the proximal end side link 61 and the distal end portion of the distal end side link 62. Because this imaginary straight line extends in the reference direction D as described above, the distal end portion of the distal end side link 62 moves away from the first axis L1 in the reference direction D. In addition, due to the configuration with the above pulley ratio, the distal end portion of the distal end side link 62 moves linearly in the reference direction D. As a result, the instrument 42 inserted into the sleeve 110 can be smoothly moved in the reference direction D.

By causing the first translation arm drive shaft 37 and the second translation arm drive shaft 38 to synchronously pivot, it is possible to rotate the entire translation arm 35 around the first axis L1 with respect to the arm body 30. This can change the reference direction D and the direction in which the distal end portion of the distal end side link 62 moves linearly to a direction around the first axis L1. In addition, the first pulley 65a may be fixed to the distal end portion 30a of the arm body 30 without providing the second translation arm drive shaft 38 and the translation arm rotation drive unit 48, and the entire translation arm 35 may be rotated around the first axis L1 by moving the distal end portion of the arm body 30.

Due to the rotation of the distal end side link 62, the fourth pulley 65d then revolves around the second axis L2. The angular position of the third pulley 65c fixed to the distal end portion of the proximal end side link 61 by the first coupling shaft 63 does not change due to the pivoting operation of the distal end side link 62. In addition, because the third pulley 65c and the fourth pulley 65d are coupled by the second belt 67b, the fourth pulley 65d rotates in the opposite direction to the rotation direction of the distal end side link 62 with respect to the third pulley 65c, resulting in a change in phase between the third pulley 65c and the fourth pulley 65d. The angular displacement is then α° based on the pulley ratio. When the fourth pulley 65d rotates, the pivot shaft 68 fixed to the fourth pulley 65d rotates, and the holder 36 fixed to the pivot shaft 68 rotates through α° around the third axis L3. As a result, the holder 36 moves away from the first axis L1 in the reference direction D while maintaining its posture with respect to the arm body 30.

Similarly, when the first translation arm drive shaft 37 is differentially operated with respect to second translation arm drive shaft 38 and rotated in the R2 direction (direction opposite to R1), the proximal end side link 61 pivots from the advancement angular position P2 toward the retraction angular position P1, and the distal end side link 62 pivots from the opening angular position P4 toward the closing angular position P3. As a result, the holder 36 moves toward the first axis L1 in the reference direction D while maintaining its posture with respect to the arm body 30.

As described above, the translation arm 35 can move (advance/retract or reciprocate) the instrument 42 in the reference direction D by shifting between a folded state in which the proximal end side link 61 is located at the retraction angular position P1 and the distal end side link 62 is located at the closing angular position P3 and an extended state in which the proximal end side link 61 is located at the advancement angular position P2 and the distal end side link 62 is located at the opening angular position P4. Accordingly, as compared with the configuration in which the holder 36 is moved in the reference direction D by a prismatic joint, the dimensions of each arm 3 in the reference direction D can be made compact.

The translation arm 35 is connected to the arm body 30 and the holder 36 by pivot joints, and links constituting the translation arm 35, that is, the proximal end side link 61 and the distal end side link 62, are also connected by rotary joints. Accordingly, because the exposed portion of the inner space of the arm to the outer space can be reduced, it is possible to effectively restrict scattering of dust, germs, and the like generated in the inner space of the arm to the outer space. In addition, as described above, using the seal bearing for the rotary joints makes it possible to prevent dust, germs, and the like generated in the inner space of the arm from scattering to the outer space. This makes it possible to effectively prevent contamination of the operating room.

Because the interlocking mechanism 64 is configured to cause the distal end side link 62 to pivot in conjunction with the pivoting operation of the proximal end side link 61, it is possible to omit the drive unit that causes only the distal end side link 62 to pivot and simplify the configuration of the translation arm 35.

A mechanism using a timing belt has been described as an example of the interlocking mechanism 64. Instead, for example, a mechanism including a gear train may be used, and a known link mechanism may be used. In addition, the proximal end side link 61 and the distal end side link 62 may be configured to pivot by using driving forces from separately provided drive units.

### [First Modification of Translation Arm]

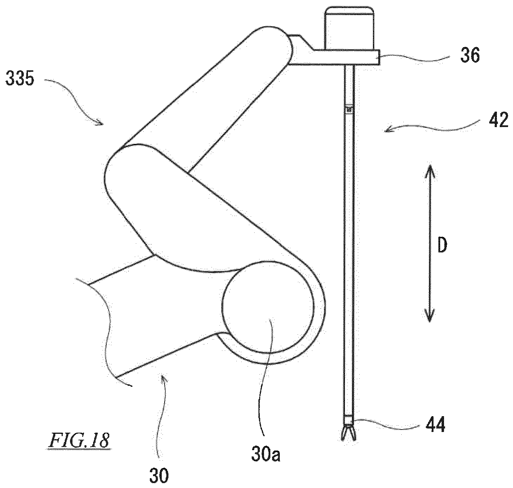

Fig. 18 is a view showing the first modification of each translation arm. In the above embodiment, the translation arm 35 is disposed so as to be located on the side from the proximal end portion toward the distal end portion of the shaft 43 of the instrument 42 held by the holder 36 in the reference direction D from the distal end portion 30a of the arm body 30. However, this is not exhaustive. Instead of this configuration, the translation arm 35 as the translation arm 335 may be disposed so as to be located on the side from the distal end portion toward the proximal end portion of the shaft 43 of the instrument 42 held by the holder 36 in the reference direction D from the distal end portion 30a of the arm body 30.

### [Second Modification of Translation Arm]

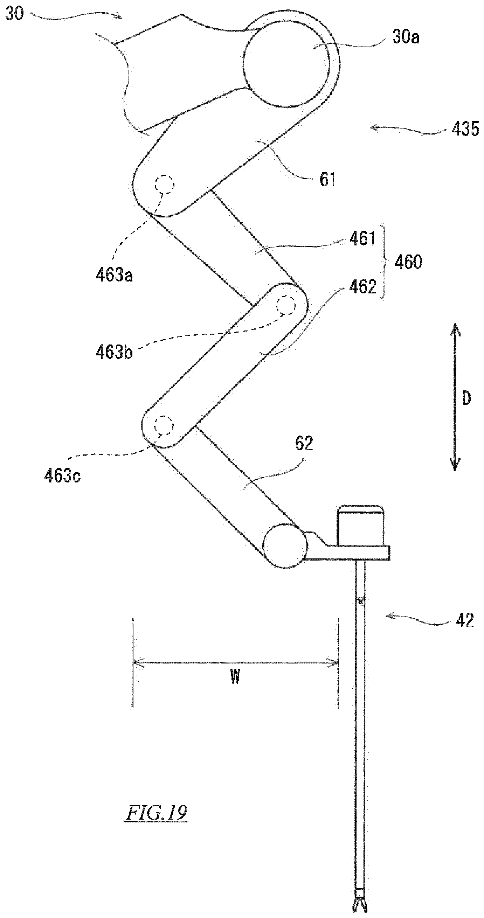

Fig. 19 is a view showing the second modification of the translation arm. The above embodiment has exemplified the configuration of the translation arm 35 in which the proximal end portion of the distal end side link 62 is attached to the distal end portion of the proximal end side link 61. However, this is not exhaustive. Instead of this, the translation arm may have the following configuration..

That is, the translation arm 435 is configured such that an intermediate link unit 460 having a first intermediate link 461 and a second intermediate link 462 is interposed between the proximal end side link 61 and the distal end side link 62. In addition, the translation arm 435 includes a first coupling shaft 463a coupling the proximal end side link 61 and the first intermediate link 461, a second coupling shaft 463b coupling the first intermediate link 461 and the second intermediate link 462, and a third coupling shaft 463c coupling the second intermediate link 462 and the distal end side link 62.

The interlocking mechanism of the translation arm 435 (not shown) is configured to cause the first intermediate link 461 to pivot about the axis of the first coupling shaft 463a so as to increase the joint angle formed by the proximal end side link 61 and the first intermediate link 461, cause the second intermediate link 462 to pivot about the axis of the second coupling shaft 463b so as to increase the joint angle formed by the first intermediate link 461 and the second intermediate link 462, and cause the distal end side link 62 to pivot about the axis of the third coupling shaft 463c so as to increase the joint angle formed by the second intermediate link 462 and the distal end side link 62 in conjunction with the pivoting operation of the proximal end side link 61 from the retraction angular position P1 to the advancement angular position P2.

This makes it possible to increase the moving distance of the instrument 42. In addition, it is possible to reduce a width dimension W of the translation arm 35, that is, the dimension of the translation arm 35 in a direction orthogonal to the reference direction D and the first axis L1.

### [Configuration Example of Swing Mechanism]

As shown in Fig. 7, each arm 3 is provided with a swing mechanism 46. The swing mechanism 46 is a mechanism that is interposed between the arm 3 and the end effector 44 and swings the distal end portion of the shaft 43 and the end effector 44 in the radial direction centered on the reference direction D.

The swing mechanism 46 is provided, for example, at an intermediate portion of the shaft 43. That is, the shaft 43 is divided into a proximal end side member 43a and a distal end side member 43b, and the swing mechanism 46 is interposed between them. A mechanism using a universal joint having two rotational degrees of freedom will be described as an example of the swing mechanism 46.

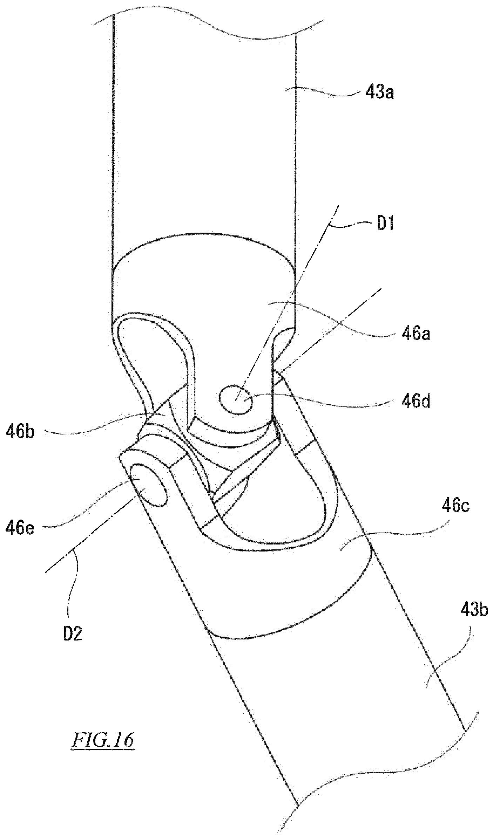

Fig. 16 is a diagram showing a configuration example of the swing mechanism 46.

The swing mechanism 46 includes a first member 46a, a second member 46b, and a third member 46c. The first member 46a is fixed to the proximal end side member 43a. The second member 46b is coupled to the first member 46a by a first pivot shaft 46d extending in a first direction D1 orthogonal to the reference direction D and is attached to the first member 46a so as to be pivotal about the axis of the first pivot shaft 46d. The third member 46c is coupled to the second member 46b by a second pivot shaft 46e extending in a second direction D2 orthogonal to the first direction D1 and is attached to the second member 46b so as to be pivotal about the axis of the second pivot shaft 46e.

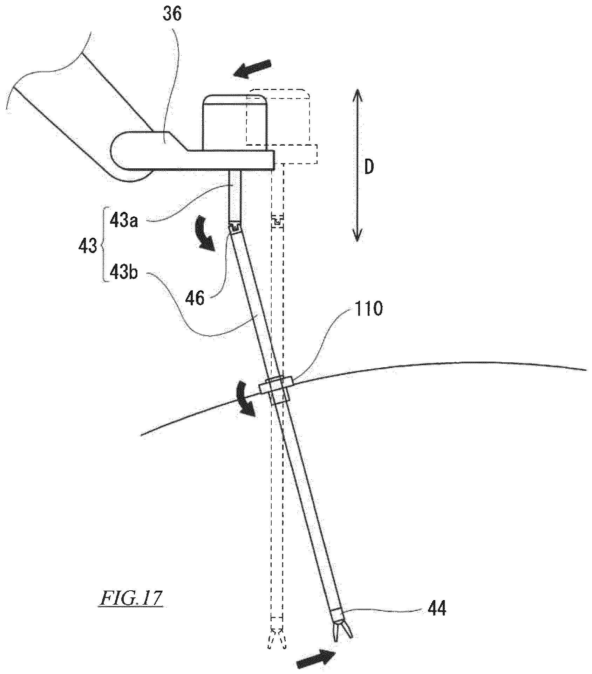

Fig. 17 is a diagram showing an operation example of the swing mechanism 46.

An operation example of the swing mechanism 46 will be described below with reference to Fig. 17.

The end effector 44 of the instrument 42 is introduced into the body of the patient P via the sleeve 110 indwelled in the incision of the body surface of the patient P. In the initial state indicated by the broken line in Fig. 17, the sleeve 110 is located in the reference direction D.

Subsequently, when the arm 3 moves the holder 36 in a direction intersecting the reference direction D, the distal end side member 43b of the shaft 43 swings around the sleeve 110, and the distal end portion of the shaft 43 and the end effector 44 move in the direction opposite to the moving direction of the holder 36. In addition, when the distal end side member 43b of the shaft 43 swings, the sleeve 110 through which the shaft 43 extends swings together with the distal end side member 43b so as to be oriented in the extending direction of the distal end side member 43b.

At this time, when the sleeve 110 departs from the axis of the proximal end side member 43a, the distal end side member 43b of the shaft 43 swings in the radial direction centered on the axis of the proximal end side member 43a, that is, in the radial direction centered on the reference direction D, such that the extending direction of the distal end side member 43b of the shaft 43 coincides with the direction of the insertion hole of the sleeve 110. This can prevent the sleeve 110 dwelled in the patient P from being towed in the moving direction of the holder 36, and hence can alleviate damage given to the incision of the patient P.

### [First Modification of Swing Mechanism]

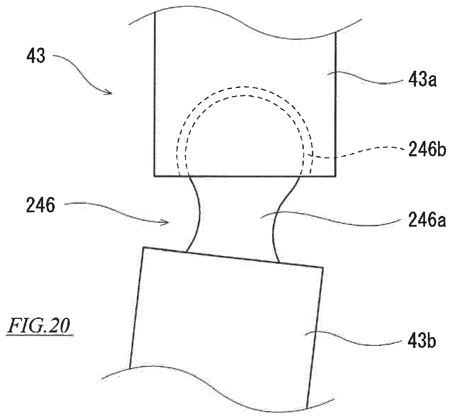

Fig. 20 is a view showing the first modification of the swing mechanism. The swing mechanism may be a mechanism using a ball joint.

That is, a swing mechanism 246 has a ball portion 246a having a spherical free end and attached to the proximal end portion of the distal end side member 43b of the shaft 43 and a socket 246b having an arm shape and attached to the distal end side portion of proximal end side member 43a of the shaft 43. The inner circumferential surface of the socket 246b forms a substantially hemispherical surface. The shape of the inner circumferential surface of the socket 246b is almost the same as that of the outer circumferential surface of the ball portion 246a. As a result, the outer circumferential surface of the ball portion 246a and the inner circumferential surface of the socket 246b form a spherical surface kinematic pair, and the proximal end side member 43a and the distal end side member 43b of the shaft 43 are coupled to each other so as to have two rotational degrees of freedom.

### [Second Modification of Swing Mechanism]

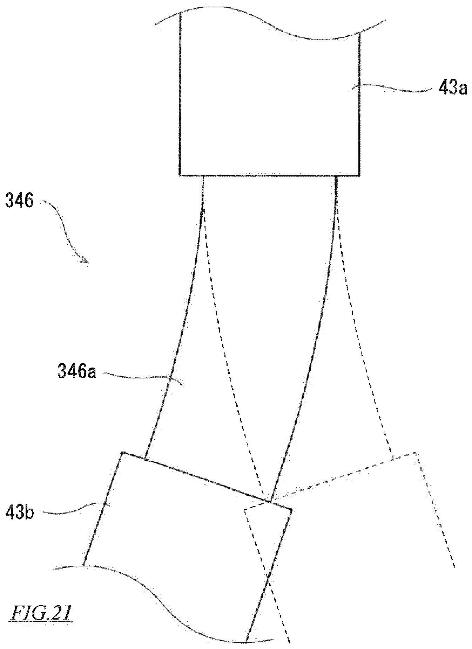

Fig. 21 is a view showing the second modification of the swing mechanism. The swing mechanism may be a mechanism using a member having elasticity.

That is, a swing mechanism 346 has an elastic portion 346a that connects the proximal end side member 43a and the distal end side member 43b. By elastically deforming the elastic portion 346a, the proximal end side member 43a and the distal end side member 43b of the shaft 43 are swingably coupled with at least two rotational degrees of freedom.

### [First Modification of Arm Having Swing Mechanism]

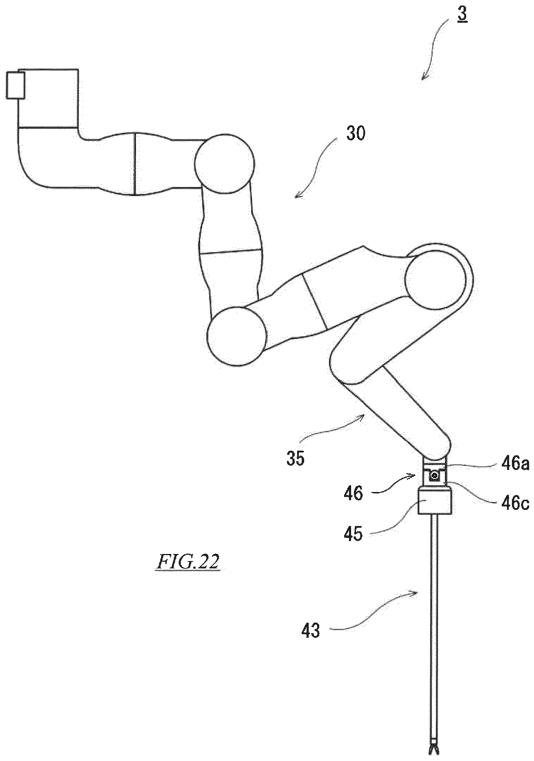

Fig. 22 is a view showing the first modification of each arm including the swing mechanism. In the above embodiment, a configuration example in which the swing mechanism 46 is provided at the intermediate portion of the shaft 43 has been described.
Alternatively, the swing mechanism 46 may be interposed between the distal end portion of the translation arm 35 and the instrument 42.

That is, the first member 46a of the swing mechanism 46 is directly attached to the distal end portion of the distal end side link 62, and the third member 46c is directly attached to the instrument 42.

### [Second Modification of Arm Having Swing Mechanism]

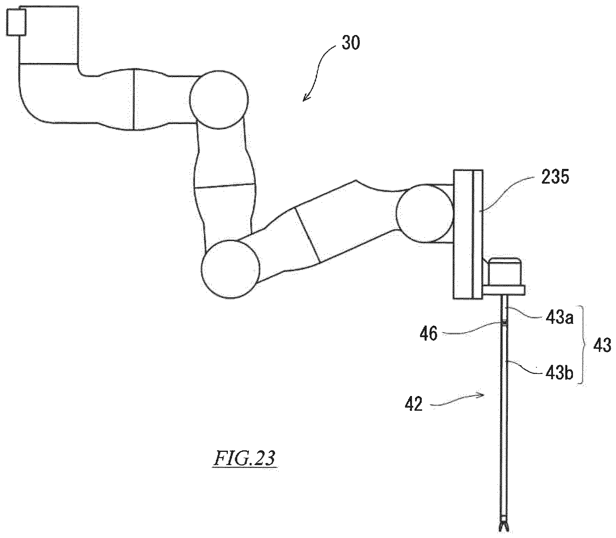

Fig. 23 is a view showing the second modification of each arm including the swing mechanism. In the first embodiment, the swing mechanism 46 is applied to the surgical system 100 including the arm 3 having the translation arm 35. Alternatively, the swing mechanism 46 may be applied to an arm having a prismatic joint 235.

### [General Overview]

As described above, the surgical system 100 (especially, the patient-side system 1) according to this embodiment includes a plurality of instruments 42 for performing a surgical operation, a platform 5, a plurality of arms 3 having proximal end portions detachably attached to the platform 5 and distal end portions, to each of which one of the plurality of instruments 42 is detachably attached, and configured to movably support the instrument 42, and an attachment lock mechanism 94 configured to lock and unlock each arm 3 attached to the platform 5. Note that the arm 3 to which the endoscope assembly 41 is attached in place of the instrument 42 is also included in the surgical system 100.

The arm 3 included in the surgical system 100 according to this embodiment is attached to a support body (that is, the platform 5 or an assembly including the platform 5 and the positioner 7), and the instrument 42 for performing a surgical operation is attached to the distal end portion of the arm 3. The arm 3 includes the base 80, at least one of the links 81 to 86 coupled in series with the base 80, and an I/F unit 801 provided at the proximal end portion of the base 80 and including the support body side engagement portion 94a provided on the support body and the arm side engagement portion 94b that can be engaged with the support body side engagement portion 94a. The I/F unit 801 is locked while being attached to the support body by the engagement between the support body side engagement portion 94a and the arm side engagement portion 94b, and the I/F unit 801 can be separated from the support body by disengagement between the support body side engagement portion 94a and the arm side engagement portion 94b.

The manipulator arm support body S included in the surgical system 100 according to this embodiment includes the platform 5 on which the arm 3 for movably supporting the instrument 42 for performing a surgical operation is mounted, and the positioner 7 for movably supporting the platform 5. The platform 5 includes a plurality of attachment ports 55 (arm attachment portions) to which the arms 3 are detachably attached and the support body side engagement portion 94a engageable with the arm side engagement portion 94b provided on each arm 3. The arm 3 is locked while being attached to the attachment port 55 by the engagement between the support body side engagement portion 94a and the arm side engagement portion 94b. The arm 3 can be unlocked so as to be separated from the attachment port 55 by disengagement between the support body side engagement portion 94a and the arm side engagement portion 94b.

According to the surgical system 100, each arm 3, and the manipulator arm support body S, the arm 3 is set on the platform 5 and is locked by the attachment lock mechanism 94 (the support body side engagement portion 94a and the arm side engagement portion 94b), thereby attaching the arm 3 to the platform 5. In addition, by unlocking the arm 3 attached to the platform 5, it is possible to detach the arm 3 from the platform 5. In this way, the arm 3 is detachable from the platform 5, and the arm 3 attached to the platform 5 can be replaced or the arm 3 attached to a certain attachment port 55 of the platform 5 can be replaced to another attachment port 55 before and/or during surgery. In this manner, before and/or during surgery, the surgical system 100 can be properly customized for the surgery by attaching the arms 3 matching the number, type, and combination set in accordance with the contents of surgery, to the platform 5.

In the conventional systems as described in PTLs 1 and 2, the configuration of the manipulator (manipulator arm and robot arm) of the surgical system is determined at the time of installation of the system. Although it is possible to change instruments before and/or during surgery, it was not supposed to change manipulators that support the instruments.

In the surgical system 100 according to this embodiment, the platform 5 has more attachment ports 55 (manipulator arm attachment portions) than the plurality of arms 3.

This makes it possible to select the attachment position of the arm 3 from the plurality of attachment ports 55 and properly customize the surgical system 100 for the surgical operation.

The surgical system 100 according to this embodiment includes the positioner 7 that movably supports the platform 5.

This can move the plurality of arms 3 to desired positions comprehensively.

The surgical system 100 according to this embodiment further includes the detection sensor 57 for detecting the presence or absence of the arm 3 attached to the platform 5 and the controller 6 (in particular, the arm management unit 603) (the management device) for detecting that the arm 3 is attached to the platform 5 based on a detection signal from the detection sensor 57.

This allows the surgical system 100 to self-check that the arm 3 is attached to the platform 5 and that the attachment is normal.

In the surgical system 100 according to this embodiment, the reader/writer 93 (reader) for reading out information held by the arm 3 is provided on the platform 5. Upon detecting that the arm 3 is attached to the platform 5, the arm management unit 603 causes the reader/writer 93 to read information, acquires the information read out by the reader/writer 93, and temporarily stores the acquired information in association with attachment position information on the platform 5.

In this way, the arm 3 owns its own information. Accordingly, it is possible to use the arm 3 upon attaching it to the platform 5 of another patient-side system 1 and to share one arm 3 among a plurality of patient-side systems 1.

In the surgical system 100 according to this embodiment, the information held by each arm 3 includes the individual identification information of the arm, and surgical information including a combination of individual identification information of the plurality of arms 3 used in surgery is set in the arm management unit 603 in advance. The arm management unit 603 outputs a warning if a combination of the individual identification information included in the information acquired from the reader/writer 93 does not correspond to the surgical information. Note that the operator O may set and input surgical information to the arm management unit 603 (controller 6) via the operation device 2.

This makes it possible to avoid performing surgery with an improper combination of arms 3.

Note that in the above surgical system 100, the information held by each arm 3 may include the individual identification information of the arm 3, and surgical information including a combination of the individual identification information of the arm 3 used in surgery and the attachment position on the platform 5 to which the arm 3 is to be attached may be set in the arm management unit 603 in advance. In this case, the arm management unit 603 outputs a warning if a combination of the individual identification information included in the information acquired from the reader/writer 93 and the attachment position on the platform 5 does not correspond to the surgical information.

With this operation, a warning is issued if the proper arm 3 is not attached to the proper attachment port 55 (that is, the preset attachment port 55). This can prevent surgery from being performed while the arm 3 is attached to the improper attachment port 55 and/or while the improper arm 3 is attached to the attachment port 55.

In the above surgical system 100, the information held by each arm 3 may include the model number information of the arm 3, and surgical information including a combination of the model number information of the arm 3 used in surgery and the attachment position on the platform 5 to which the arm 3 is to be attached may be set in the arm management unit 603 in advance. In this case, the arm management unit 603 outputs a warning if a combination of the model number information included in the information acquired from the reader/writer 93 and the attachment position on the platform 5 does not correspond to the surgical information.

Alternatively, in the above surgical system 100, the information held by each arm 3 may include the individual identification information of the arm 3, and surgical information including a combination of the model number information of the arm 3 used in surgery and the attachment position on the platform 5 to which the arm 3 is to be attached may be set in the arm management unit 603 in advance. In this case, the arm management unit 603 has a model number storage unit that stores the model number information of each arm 3 associated with individual identification information. Based on the individual identification information included in the information acquired from the reader/writer 93, the arm management unit 603 reads out the corresponding model number information from the model number storage unit, and outputs a warning if a combination of the read model number information and the attachment position on the platform 5 does not correspond to the surgery information.

In any of the configurations described above, a warning is issued if the arm 3 with a proper model number is not attached to the proper attachment port 55 (that is, the preset attachment port 55). This can prevent surgery from being performed while the arm 3 is attached to the improper attachment port 55 and/or while the arm 3 with an improper model number is attached to the attachment port 55.

In the surgical system 100 according to this embodiment, the information held by each arm 3 includes the individual identification information and the usage count information of the arm 3. The arm management unit 603 has a usage limit number storage unit that stores the usage limit number associated with individual identification information. Based on the individual identification information included in the information acquired from the reader/writer 93, the arm management unit 603 reads out the usage limit number of the arm 3 from the usage limit number storage unit, and outputs a warning if the usage count information included in the information acquired from the reader/writer 93 exceeds the usage limit number.

With this operation, a warning is issued when the arm 3 is tried to be used beyond the usage limit number, and hence it is possible to prevent the arm 3 from being used beyond the usage limit number. Note that the arm 3 that has exceeded the usage limit number may be discarded.

In the surgical system 100 according to this embodiment, the information held by each arm 3 is stored in the IC tag 91 provided for the arm 3, and the reader/writer 93 has a function of writing to the IC tag 91. The arm management unit 603 then causes the reader/writer 93 to operate so as to write, in the IC tag 91, new usage count information obtained by adding 1 to the usage count information acquired from the reader/writer 93.

With this operation, the usage count information of each arm 3 is updated every time it is used, and hence it is possible to prevent the arm 3 from being used beyond the usage limit number. In addition, because the arm 3 itself holds the usage count information of the arm 3, the arm 3 can be shared with other patient side systems 1.

In the surgical system 100 according to this embodiment, the I/F unit 801 of each arm 3 is provided with the connector 92 for connecting the wiring passed through the platform 5 (support body) and the wiring passed through the arm 3. More specifically, the socket 56 of an electric wire or communication wiring extending through the platform 5 is provided for the platform 5. When the arm 3 is attached to the platform 5 at the proximal end portion of the arm 3, the connector 92 connected to an electric wire or communication wiring of the arm 3 is provided at a position where the connector 92 is connected to the socket 56.

With this configuration, when the arm 3 is attached to the platform 5, the connector 92 and the socket 56 are connected, and the wiring system straddling the platform 5 and the arm 3 is connected.

In the surgical system 100 according to the above-described embodiment, the I/F unit 801 of each arm 3 may further be provided with an input unit that accepts an input from a power source (for example, the servomotor M31) provided for the platform 5. More specifically, in the surgical system 100, the servomotor M31 is provided for the platform 5, and the input shaft 95 to the power system of the arm 3 is provided at the proximal end portion of the arm 3. The shaft coupling 97 that connects the output shaft 96 of the servomotor M31 and the input shaft 95 so as to allow power transmission when the arm 3 is attached to the platform 5 may be provided at an end portion of the output shaft 96 and an end portion of the input shaft 95.

With this configuration, when the arm 3 is attached to the platform 5, the input shaft 95 to the power system of the arm 3 is connected to the output shaft 96 of the servomotor M31, and the power transmission system straddling the platform 5 and the arm 3 is connected.

In the surgical system 100 according to this embodiment, the platform 5, the arms 3, and the instruments 42 are installed in a sterile field, and the platform 5 may be shielded from the sterile field by the sterile drape 9.

In the technique disclosed in PTL 2, the sterile drape covers almost all the mechanical elements from the proximal end portion to the distal end portion of the manipulator assembly, and the manipulator assembly is not sterilized before and after the surgery. The sterile drape is a bag-like member made of a material selected from the group consisting of polyethylene, polyurethane, and polycarbonate, which is sufficiently larger than the outer shape of the manipulator assembly to permit movement of the manipulator inside the drape. It is difficult to specify the behavior of such a sterile drape, and it is not undeniable that the sterile drape hinders the normal operation of the manipulator assembly and the operation of the assistant.

Accordingly, in the above embodiment, each arm 3 and each instrument 42 are exposed to the sterile field, and the platform 5 is installed in the sterile field, but is shielded from the sterile field by the sterile drape 9. As described above, because the arm 3 can be detached from the platform 5, the arm 3 detached from the platform 5 can be reused after being cleaned and sterilized. Because the arm 3 can thus be exposed to the sterile field without being covered with the sterile drape 9, the operation of the arm 3 is not disturbed by the behavior of the sterile drape 9.

In the above surgical system 100, because the cleaned and sterilized clean arm 3 is used for every surgery, it is possible to restrain the propagation of bacteria and reduce residual protein in the arm 3. In principle, the arm 3 detached from the platform 5 is subjected to a cleaning process and a sterilization process, but a disinfection process may be applied in place of or in addition to the sterilization process.

### Reference Signs List

- 100: surgical system
- 1: patient-side system
- 2: operation device
- 3: patient-side manipulator arm (arm)
- 3A: camera arm
- 3B: instrument arm
- 5: platform (manipulator arm support body or its part)
- 6: controller
- 7: positioner (part of manipulator arm support body)
- 9: sterile drape
- 30: arm body
- 35: translation arm
- 36: holder
- 41: endoscope assembly
- 42: instrument
- 55: attachment port (example of manipulator arm attachment portion)
- 56: socket
- 57: detection sensor
- 91: IC tag (example of information holding unit)
- 92: connector
- 93: reader/writer (reader)
- 94: attachment lock mechanism
- 94a: support body side engagement portion
- 94b: arm side engagement portion
- 95: input shaft
- 96: output shaft
- 97: shaft coupling
- O: operator
- P: patient
- S: manipulator arm support body

## Claims

1. A surgical system (100) comprising:
a plurality of instruments (42) for performing a surgical operation;
a platform (5) having a plurality of attachment ports (55);
a plurality of manipulator arms(3) configured to movably support a plurality of instruments (42), each of the manipulator arms (3) having a proximal end portion detachably attached to one of the plurality of attachment ports (55) and a distal end portion to which one of the plurality of instruments (42) is detachably attached to;
a detection sensor (57) configured to detect presence or absence of the manipulator arm (3) attached to the platform (5);
a plurality of IC tags (91), each of the plurality of IC tags (91) is attached to one of the plurality of manipulator arms (3) and holding information on the manipulator arm (3) attached thereto;
at least one reader (93) provided at the platform (5) and configured to read out information held by the IC tags (91); and
a management device (6) configured to detect that the manipulator arm (3) is attached to the platform (5) based on a detection signal from the detection sensor (57), and is configured to, upon detecting, cause the reader (93) to read out the information, and acquire the information read out by the reader (93), and temporarily stores the acquired information in association with the attachment position information on the platform (5).
**characterized in that**
the information held by the IC tag (91) includes individual identification information of the manipulator arm (3) attached to the IC tag (91),
surgical information including a combination of a plurality of manipulator arms (3) used in surgery is set in the management device (6) in advance, and
the management device (6) configured to output a warning if a combination of individual identification information included in information acquired from the reader (93) does not correspond to the surgery information.

2. The surgical system (100) according to claim 1, wherein
the surgical information including a combination of individual identification information of each manipulator arm(3) used in surgery and an attachment position on the platform(5) to which the manipulator arm(3) is to be attached is set in the management device(6) in advance, and
the management device (6) outputs a warning if a combination of individual identification information included in information acquired from the reader (93) and the attachment position on the platform (5) does not correspond to the surgery information.

3. The surgical system (100) according to claim 1, wherein
the information held by the manipulator arm (3) includes model number information of the manipulator arm (3),
surgical information including a combination of model number information of the manipulator arm (3) used in surgery and an attachment position on the platform (5) to which the manipulator arm (3) is to be attached is set in the management device (6) in advance, and
the management device (6) outputs a warning if a combination of model number information included in information acquired from the reader (93) and the attachment position on the platform (5) does not correspond to the surgery information.

4. The surgical system (100) according to claim 1, wherein
the information held by the manipulator arm (3) includes individual identification information of the manipulator arm (3),
surgical information including a combination of the model number information of a manipulator arm (3) to be used in surgery and an attachment position on the platform (5) to which the manipulator arm (3) is to be attached is set in the management device (6) in advance, and
the management device (6) includes a model number storage unit storing model number information of the manipulator arm (3) associated with individual identification information, reads out corresponding model number information from the model number storage unit based on the individual identification information included in the information acquired from the reader (93), and outputs a warning if a combination of the readout model number information and an attachment position on the platform (5) does not correspond to the surgery information.

5. The surgical system (100) according to any one of claims 1 to 4, wherein
the information held by the manipulator arm (3) includes individual identification information and usage count information of the manipulator arm (3), and
the management device (6) includes a usage limit number storage unit storing a usage limit number associated with the individual identification information, reads out a usage limit number of the manipulator arm (3) from the usage limit number storage unit based on the individual identification information included in the information acquired from the reader (93), and outputs a warning if the usage count information included in the information acquired from the reader (93) exceeds the usage limit number.

6. The surgical system (100) according to claim 5, wherein
the information held by the manipulator arm (3) is stored in the IC tag (91) provided for the manipulator arm (3),
the reader (93) has a function of writing to the IC tag (91), and
the management device (6) causes the reader (93) to operate so as to write, in the IC tag (91), new usage count information obtained by adding 1 to the usage count information acquired from the reader (93).

7. The surgical system (100) according to any one of claims 1 to 6, wherein the platform (5), the manipulator arm (3), and the instrument (42) are installed in a sterile field, and the platform (5) is shielded from the sterile field by a sterile drape.

## Patentansprüche

1. Chirurgisches System (100), umfassend:
eine Mehrzahl von Instrumenten (42) zum Durchführen eines chirurgischen Eingriffs;
eine Plattform (5) mit einer Mehrzahl von Anbringungsöffnungen (55);
eine Mehrzahl von Manipulatorarmen (3), die konfiguriert sind, eine Mehrzahl von Instrumenten (42) beweglich zu stützen bzw. zu tragen, wobei jeder der Manipulatorarme (3) einen proximalen Endabschnitt, der abnehmbar an einer der Mehrzahl von Anbringungsöffnungen (55) angebracht ist, und einen distalen Endabschnitt aufweist, an dem eines der Mehrzahl von Instrumenten (42) abnehmbar angebracht ist;
einen Erkennungssensor (57), der konfiguriert ist, das Vorhandensein oder Nichtvorhandensein des an der Plattform (5) angebrachten Manipulatorarms (3) zu erkennen;
eine Mehrzahl von IC-Tags (91), wobei jedes der Mehrzahl von IC-Tags (91) an einem der Mehrzahl von Manipulatorarmen (3) angebracht ist und Informationen über den daran angebrachten Manipulatorarm (3) hält;
zumindest ein Lesegerät (93), das an der Plattform (5) bereitgestellt und konfiguriert ist, Informationen auszulesen, die von den IC-Tags (91) gehalten werden; und
eine Verwaltungsvorrichtung (6), die konfiguriert ist, zu erkennen, dass der Manipulatorarm (3) an der Plattform (5) angebracht ist, und zwar basierend auf einem Erkennungssignal von dem Erkennungssensor (57), und die konfiguriert ist, beim Erkennen das Lesegerät (93) zu veranlassen, die Informationen auszulesen und die von dem Lesegerät (93) ausgelesenen Informationen zu erfassen, und die die erfassten Informationen in Verbindung mit den Anbringungspositionsinformationen auf der Plattform (5) vorübergehend speichert,
**dadurch gekennzeichnet, dass**
die von dem IC-Tag (91) gehaltenen Informationen individuelle Identifikationsinformationen des an dem IC-Tag (91) angebrachten Manipulatorarms (3) enthalten,
chirurgische Informationen, die eine Kombination einer Mehrzahl von in der Chirurgie verwendeten Manipulatorarmen (3) beinhalten, im Voraus in der Verwaltungsvorrichtung (6) eingestellt werden, und
die Verwaltungsvorrichtung (6) konfiguriert ist, eine Warnung auszugeben, wenn eine Kombination von individuellen Identifikationsinformationen, die in von dem Lesegerät (93) erfassten Informationen enthalten sind, nicht den chirurgischen Informationen entspricht.

2. Chirurgisches System (100) nach Anspruch 1, wobei
die chirurgischen Informationen, die eine Kombination aus individuellen Identifikationsinformationen jedes bei dem Eingriff verwendeten Manipulatorarms (3) und einer Anbringungsposition auf der Plattform (5), an der der Manipulatorarm (3) zu befestigen ist, beinhalten, in der Verwaltungsvorrichtung (6) im Voraus eingestellt werden, und
die Verwaltungsvorrichtung (6) eine Warnung ausgibt, wenn eine Kombination aus individuellen Identifikationsinformationen, die in den von dem Lesegerät (93) erfassten Informationen enthalten sind, und der Anbringungsposition auf der Plattform (5) nicht den chirurgischen Informationen entspricht.

3. Chirurgisches System (100) nach Anspruch 1, wobei
die von dem Manipulatorarm (3) gehaltenen Informationen Modellnummerinformationen des Manipulatorarms (3) beinhalten,
chirurgische Informationen, die eine Kombination aus Modellnummerinformationen des bei dem Eingriff verwendeten Manipulatorarms (3) und einer Anbringungsposition auf der Plattform (5), an der der Manipulatorarm (3) zu befestigen ist, beinhalten, in der Verwaltungsvorrichtung (6) im Voraus eingestellt werden, und
die Verwaltungsvorrichtung (6) eine Warnung ausgibt, wenn eine Kombination aus Modellnummerninformationen, die in den von dem Lesegerät (93) erfassten Informationen enthalten sind, und der Anbringungsposition auf der Plattform (5) nicht den chirurgischen Informationen entspricht.

4. Chirurgisches System (100) nach Anspruch 1, wobei
die von dem Manipulatorarm (3) gehaltenen Informationen individuelle Identifikationsinformationen des Manipulatorarms (3) beinhalten,
chirurgische Informationen, die eine Kombination aus den Modellnummerninformationen eines bei dem Eingriff zu verwendenden Manipulatorarms (3) und einer Anbringungsposition auf der Plattform (5), an der der Manipulatorarm (3) zu befestigen ist, beinhalten, in der Verwaltungsvorrichtung (6) im Voraus eingestellt werden, und
die Verwaltungsvorrichtung (6) eine Modellnummer-Speichereinheit enthält, die Modellnummerinformationen des Manipulatorarms (3) speichert, die mit individuellen Identifikationsinformationen verknüpft sind, entsprechende Modellnummerninformationen aus der Modellnummer-Speichereinheit basierend auf den individuellen Identifikationsinformationen ausliest, die in den von dem Lesegerät (93) erfassten Informationen enthalten sind, und eine Warnung ausgibt, wenn eine Kombination der ausgelesenen Modellnummerinformationen und einer Anbringungsposition auf der Plattform (5) nicht den chirurgischen Informationen entspricht.

5. Chirurgisches System (100) nach einem der Ansprüche 1 bis 4, wobei
die von dem Manipulatorarm (3) gehaltenen Informationen individuelle Identifikationsinformationen und Nutzungszählungsinformationen des Manipulatorarms (3) beinhalten, und
die Verwaltungsvorrichtung (6) eine Nutzungsgrenzahl-Speichereinheit enthält, die eine Nutzungsgrenzahl speichert, die mit den individuellen Identifikationsinformationen verknüpft ist, eine Nutzungsgrenzahl des Manipulatorarms (3) aus der Nutzungsgrenzahl-Speichereinheit basierend auf den individuellen Identifikationsinformationen ausliest, die in den von dem Lesegerät (93) erfassten Informationen enthalten sind, und eine Warnung ausgibt, wenn die Nutzungszählungsinformationen, die in den von dem Lesegerät (93) erfassten Informationen enthalten sind, die Nutzungsgrenzzahl überschreiten.

6. Chirurgisches System (100) nach Anspruch 5, wobei
die von dem Manipulatorarm (3) gehaltenen Informationen in dem für den Manipulatorarm (3) bereitgestellten IC-Tag (91) gespeichert sind,
das Lesegerät (93) eine Funktion des Schreibens auf das IC-Etikett (91) aufweist, und
die Verwaltungsvorrichtung (6) das Lesegerät (93) veranlasst, dahingehend zu arbeiten, neue Nutzungszählungsinformationen in das IC-Tag (91) zu schreiben, die durch Addieren von 1 zu den Nutzungszählungsinformationen, die von dem Lesegerät (93) erfasst werden, erhalten werden.

7. Chirurgisches System (100) nach einem der Ansprüche 1 bis 6, wobei die Plattform (5), der Manipulatorarm (3) und das Instrument (42) in einem sterilen Feld bzw. Bereich installiert sind und die Plattform (5) durch ein steriles Tuch von dem sterilen Feld abgeschirmt ist.

## Revendications

1. Système chirurgical (100) comprenant :
une pluralité d'instruments (42) pour réaliser une opération chirurgicale ;
une plate-forme (5) ayant une pluralité de ports de connexion (55) ;
une pluralité de bras manipulateurs (3) configurés pour supporter de manière mobile une pluralité d'instruments (42), chacun des bras manipulateurs (3) ayant une portion d'extrémité proximale connectée de manière amovible à un de la pluralité de ports de connexion (55) et une portion d'extrémité distale à laquelle un de la pluralité d'instruments (42) est connecté de manière amovible ;
un capteur de détection (57) configuré pour détecter la présence ou l'absence du bras manipulateur (3) connecté à la plate-forme (5) ;
une pluralité d'étiquettes de CI (91), chacune de la pluralité d'étiquettes de CI (91) est connectée à un de la pluralité de bras manipulateurs (3) et détient des informations sur le bras manipulateur (3) connecté à elle ;
au moins un lecteur (93) prévu au niveau de la plate-forme (5) et configuré pour lire des informations détenues par les étiquettes de CI (91) ; et
un dispositif de gestion (6) configuré pour détecter que le bras manipulateur (3) est connecté à la plate-forme (5) en fonction d'un signal de détection provenant du capteur de détection (57), et est configuré pour, dès la détection, amener le lecteur (93) à lire les informations, et acquérir les informations lues par le lecteur (93), et stocke temporairement les informations acquises en association avec les informations de position de connexion sur la plate-forme (5), **caractérisé en ce que**
les informations détenues par l'étiquette de CI (91) incluent des informations d'identification individuelle du bras manipulateur (3) connecté à l'étiquette de CI (91),
des informations chirurgicales incluant une combinaison d'une pluralité de bras manipulateurs (3) utilisés en chirurgie sont réglées dans le dispositif de gestion (6) en avance, et
le dispositif de gestion (6) configuré pour émettre un avertissement si une combinaison d'informations d'identification individuelle incluses dans des informations acquises auprès du lecteur (93) ne correspond pas aux informations chirurgicales.

2. Système chirurgical (100) selon la revendication 1, dans lequel
les informations chirurgicales incluant une combinaison d'informations d'identification individuelle de chaque bras manipulateur (3) utilisé en chirurgie et d'une position de connexion sur la plate-forme (5) à laquelle le bras manipulateur (5) doit être connecté sont réglées dans le dispositif de gestion (6) en avance, et
le dispositif de gestion (6) émet un avertissement si une combinaison d'informations d'identification individuelle incluses dans des informations acquises auprès du lecteur (93) et de la position de connexion sur la plate-forme (5) ne correspond pas aux informations chirurgicales.

3. Système chirurgical (100) selon la revendication 1, dans lequel
les informations détenues par le bras manipulateur (3) incluent des informations de numéro de modèle du bras manipulateur (3),
des informations chirurgicales incluant une combinaison d'informations de numéro de modèle du bras manipulateur (3) utilisé en chirurgie et d'une position de connexion sur la plate-forme (5) à laquelle le bras manipulateur (5) doit être connecté sont réglées dans le dispositif de gestion (6) en avance, et
le dispositif de gestion (6) émet un avertissement si une combinaison d'informations de numéro de modèle incluses dans des informations acquises auprès du lecteur (93) et de la position de connexion sur la plate-forme (5) ne correspond pas aux informations chirurgicales.

4. Système chirurgical (100) selon la revendication 1, dans lequel
les informations détenues par le bras manipulateur (3) incluent des informations d'identification individuelle du bras manipulateur (3),
des informations chirurgicales incluant une combinaison des informations de numéro de modèle d'un bras manipulateur (3) devant être utilisé en chirurgie et d'une position de connexion sur la plate-forme (5) à laquelle le bras manipulateur (5) doit être connecté sont réglées dans le dispositif de gestion (6) en avance, et
le dispositif de gestion (6) inclut un module de stockage de numéro de modèle stockant des informations de numéro de modèle du bras manipulateur (3) associées à des informations d'identification individuelle, lit des informations de numéro de modèle correspondantes depuis le module de stockage de numéro de modèle en fonction des informations d'identification individuelle incluses dans les informations acquises auprès du lecteur (93), et émet un avertissement si une combinaison des informations de numéro de modèle lues et d'une position de connexion sur la plate-forme (5) ne correspond pas aux informations chirurgicales.

5. Système chirurgical (100) selon l'une quelconque des revendications 1 à 4, dans lequel
les informations détenues par le bras manipulateur (3) incluent des informations d'identification individuelle et des informations de compte d'usages du bras manipulateur (3), et
le dispositif de gestion (6) inclut un module de stockage de nombre limite d'usages stockant un nombre limite d'usages associé aux informations d'identification individuelle, lit un nombre limite d'usages du bras manipulateur (3) depuis le module de stockage de nombre limite d'usages en fonction des informations d'identification individuelle incluses dans les informations acquises auprès du lecteur (93), et émet un avertissement si les informations de compte d'usages incluses dans les informations acquises auprès du lecteur (93) dépassent le nombre limite d'usages.

6. Système chirurgical (100) selon la revendication 5, dans lequel
les informations détenues par le bras manipulateur (3) sont stockées dans l'étiquette de CI (91) prévue pour le bras manipulateur (3),
le lecteur (93) a une fonction d'écriture sur l'étiquette de CI (91), et
le dispositif de gestion (6) amène le lecteur (93) à opérer de manière à écrire, dans l'étiquette de CI (91), de nouvelles informations de compte d'usages obtenues en ajoutant 1 aux informations de compte d'usages acquises auprès du lecteur (93).

7. Système chirurgical (100) selon l'une quelconque des revendications 1 à 6, dans lequel la plate-forme (5), le bras manipulateur (3) et l'instrument (42) sont installés dans un champ stérile, et la plate-forme (5) est protégée du champ stérile par un drap stérile.
